# EUROPEAN PATENT APPLICATION

(11) **EP 3 415 166 A1**
(43) Date of publication of application: **19.12.2018**
(21) Application number: 18176143.8
(22) Date of filing: 31.10.2013
(51) Int. Cl.: A61K 48/00, A61K 35/34, A61P 9/00, A61K 38/16, A61N 5/06

(54) **LIGHT-SENSITIVE ION CHANNELS FOR INDUCTION OF CARDIAC ACTIVITY**

(30) Priority: 01.11.2012 US 201261721053 P
(62) Divisional of application: 13850708.2
(71) Applicant: Rappaport Family Institute for Research in the Medical Sciences, Haifa 3109602 (IL)
(72) Inventor: NUSSINOVITCH, Udi, 49213 Petach Tikva (IL); GEPSTEIN, Lior, 3475310 Haifa (IL)
(74) Representative: Pearl Cohen Zedek Latzer Baratz UK LLP

(57) **Abstract**

A pharmaceutical composition comprising a cell transfected with a gene encoding a light-sensitive channel for use in the treatment of a disease or disorder associated with suppressed, slow, irregular or unsynchronized cardiac activity in a human patient upon exposure of the composition to light after the composition is introduced by direct injection into a plurality of sites of contractile tissue in the heart of the patient.

## Description

### FIELD OF THE INVENTION

The present invention relates to methods, kits and pharmaceutical compositions for inducing a controlled cardiac activity, thereby treating cardiac diseases and disorders associated with reduced, suppressed, or unsynchronized cardiac activity.

### BACKGROUND OF THE INVENTION

Electronic cardiac pacemakers have been successfully implanted since the 1960's, however, their use is limited by the number of wires and electrodes that can be implanted in the myocardium.

Bacterial light sensitive rhodopsins, also known as bacteriorhodopsins, correspond to light-sensitive proton-pump, isolated from halobacterium, known in the art since the 1970's. Channelrhodopsin, a non-selective light-sensitive cation channel (Yizhar et al. Neuron, 71:9-34, 2011) was found in green-alga Chlamydomonas. The utilization of Channelorhodopsin-2 (ChR2; Nagel, et al. PNAS, 2003, 100(24), 13940-13945) was applied for neural excitation in mammalian cells and was also used for influencing cardiac excitability.

Arrenberg et al. (Science, 2010, 330: 971-4) disclose zebrafish cardiomyocytes expressing halorhodopsin and channelrhodopsin.

Bruegmann et al. (Nat. Methods 2010; 7: 897-900) disclose transgenic mouse embryonic stem cells expressing ChR2, cardiomyocytes differentiated therefrom and transgenic mice generated from said embryonic stem cells.

Abilez et al. (Biophys J., 2011; 101: 1326-34) disclose human induced pluripotent embryonic stem cell-derived cardiomyocytes stably transfected with ChR2 or with halorhodopsin.

Jia et al. (Circ. Arrhythm Electrophysiol., 2011; 4: 753-60) disclose a stable ChR2-expressing HEK cell line.

Abilez O.J. (Cardiac Optogenetics,2012, 34th Annual International Conference of the IEEE EMBS, 1386-1389) discloses a transduced human induced pluripotent stem cell (hiPSC) line expressing ChR2 and NpHR1.0.

There is an unmet need for methods directed to controlled induction and improvement of cardiac activity in vivo, with minimal invasiveness and side effects.

### SUMMARY OF THE INVENTION

The present invention provides methods for treating heart conditions manifested in aberrant, irregular, and particularly suppressed or non-simultaneous contraction of the heart. Advantageously, the methods of the invention are suitable for treating the heart at one or more specific sites or areas rather than the entire heart. Furthermore, as demonstrated in the present invention, the methods of the invention are directed to cure improper contraction of the heart by inducing a predetermined, synchronous heart rate.

The present invention stems in part from the finding that the heart rate can be induced and maintained at proper (normal), pre-determined rates when only one or a few selected site(s) in the heart are transformed, by gene or cell therapy. According to some embodiments, said pre-selected site(s) corresponds to one or more loci diagnosed with suppressed cardiac activity.

Thus, in one aspect, the present invention provides a method for treating a disease or disorder associated with suppressed, slow, irregular or unsynchronized cardiac activity in a patient in need thereof, comprising the steps of introducing into at least one site of a contractile tissue in the heart of said patient a pharmaceutical composition comprising a gene encoding a light-sensitive channel; and exposing said at least one site to light thereby inducing a controlled normal heart electrical activity in said patient.

The present invention further provides, in an aspect, a method for treating a disease or disorder associated with suppressed, slow, irregular or unsynchronized cardiac activity in a patient in need thereof, comprising the steps of introducing into at least one site of a contractile tissue in the heart of said patient a pharmaceutical composition comprising at least one cell transfected with a gene encoding a light-sensitive channel; and exposing said at least one site to light thereby inducing a controlled, normal, heart electrical activity in said patient.

The present invention further provides, in an aspect, a pharmaceutical composition comprising a gene encoding a light-sensitive channel for the treatment of a disease or disorder associated with suppressed, slow, irregular or unsynchronized cardiac activity upon exposure of said composition to light after said composition is introduced into at least one site of a contractile tissue in a heart.

The present invention further provides, in an aspect, a pharmaceutical composition comprising at least one cell transfected with a gene encoding a light-sensitive channel for the treatment of a disease or disorder associated with suppressed, slow, irregular or unsynchronized cardiac activity, upon exposure of said composition to light after said composition is introduced into at least one site of a contractile tissue in a heart.

The present invention further provides, in an aspect, the use of a pharmaceutical composition comprising a gene encoding a light-sensitive channel for the treatment of a disease or disorder associated with suppressed, slow, irregular or unsynchronized cardiac activity upon exposure of said composition to light after said composition is introduced into at least one site of a contractile tissue in a heart.

The present invention further provides, in an aspect, the use of a pharmaceutical composition comprising at least one cell transfected with a gene encoding a light-sensitive channel for the treatment of a disease or disorder associated with suppressed, slow, irregular or unsynchronized cardiac activity, upon exposure of said composition to light after said composition is introduced into at least one site of a contractile tissue in a heart.

In some embodiments, inducing a controlled heart electrical activity is selected from the group consisting of inducing cardiomyocytes depolarization, inducing controlled pacing, inducing controlled cardiac activation, inducing synchronized pacing, inducing synchronizing conduction, and reversing blocked or slow conduction. Each possibility represents a separate embodiment of the present invention.

In some embodiments, said disease or disorder is selected from the group consisting of asynchronous contraction of the ventricles, asynchronous contraction of the atriums, cardiac arrhythmia, bradyarrhythmia, reentrant (due to the presence of abnormal conduction) and other forms of tachyarrhythmia. Each possibility represents a separate embodiment of the present invention.

In some embodiments, said light-sensitive channel is Channelorhodopsin-2 or an active variant, derivative or fragment thereof. Each possibility represents a separate embodiment of the present invention.

In some embodiments, said light-sensitive channel is Channelorhodopsin-2.

In some embodiments, said light-sensitive channel is selected from the group consisting of ChR2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T), ChR2-step function opsins, such as, ChR2(C129), ChR2(C128A), ChR2(C128S), ChR2(D156A) and ChR2(128S/156A), VChR1-step function opsins, such as, VChR1(C123S) and VChR1(123S/151A) and active variants, derivatives or fragments thereof. Each possibility represents a separate embodiment of the present invention.

In certain such embodiments, said light-sensitive channel is selected from the group consisting of ChR2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T) and active variants, derivatives or fragments thereof. Each possibility represents a separate embodiment of the present invention.

In some embodiments, exposing the at least one site to light, depolarizes a plurality of cells in said at least one site.

In some embodiments, said at least one site at said contractile tissue is selected from the group consisting of the myocardial apex, the apical region of the heart, the sinoatrial node, the atrioventricular node, the left bundle branch, the right bundle branch, the conduction system (bundle branches and His-purkinje system), the right atrium, the left atrium, the right ventricular myocardium, the left ventricular myocardium, the right ventricle and the left ventricle. Each possibility represents a separate embodiment of the present invention.

In some embodiments, said at least one site at said contractile tissue is the myocardial apex.

In some embodiments, said at least one site at said contractile tissue is the apical region of the heart.

In some embodiments, said exposing comprises exposing a plurality of sites to light.

In some embodiments, said plurality of sites is exposed to light simultaneously.

In other embodiments, said plurality of sites is exposed to light consecutively.

In some embodiments, said light has a wavelength within the range of 350nm to 600nm.

In some embodiments, said light has a wavelength of about within the range of 450nm to 560nm.

In some embodiments, said light is delivered at an intensity of at least 7 mW/mm².

In some embodiments, said light is a flashing light.

In some embodiments, said flashing light is delivered at a frequency ranging from 60 to 300 flashes/min.

In other embodiments, said frequency is lower than 200 flashes/min.

In yet other embodiments, the duration of each flash of said flashing light is at least 1 ms.

In some embodiment, said pharmaceutical composition comprises a plurality of cells, each cell transfected with said gene encoding a light-sensitive channel

In some embodiments, exposing said at least one site to light, depolarizes said plurality of cells in said at least one site.

In some embodiments, said exposing said at least one site to light comprises exposing a plurality of sites to light, each site of said plurality of sites comprises at least one cell transfected with a gene encoding a light-sensitive channel.

In some embodiments, said cell is selected from the group consisting of fibroblasts, cardiomyocytes and derivatives stem cells, such as, embryonic stem cells and stem cells derivatives. Each possibility represents a separate embodiment of the present invention.

The term "stem cells derivatives" as used herein refers to any cells derived from stems cells, including, human progenitor cells derived from pluripotent human embryonic stem cells, such as, cardiomyocytes derived from stem cells.

In some embodiments, said cell is an autologous cell derived from said patient. In some embodiments, the cell is an autologous cell selected from the group consisting of fibroblasts, cardiomyocytes and stem cells such as embryonic stem cells. Each possibility represents a separate embodiment of the present invention.

In some embodiments, said at least one cell is capable of electronic coupling or fusing with said contractile tissue.

The present invention further provides, in an aspect, a kit for treating a disease or disorder associated with suppressed, slow, irregular or unsynchronized cardiac activity in a patient in need thereof, comprising a pharmaceutical composition comprising at least one cell transfected with a gene encoding a light-sensitive channel; and means for facilitating coupling or fusing said at least one cell with a contractile tissue of the heart of a subject in need thereof.

The present invention further provides, in an aspect, a kit for treating a disease or disorder associated with suppressed, slow, irregular or unsynchronized cardiac activity in a patient in need thereof, comprising a pharmaceutical composition comprising a gene encoding a light-sensitive channel; and means for facilitating transfecting a contractile tissue of the heart of a subject in need thereof with said gene.

In some embodiments, the kit further comprises a light source adapted for providing any one or more of the following light at a wavelength within the range of 350nm to 600 nm, light at a wavelength within the range of 450nm to 560nm, flashing light, flashing light ranging from 60 to 300 flashes/min, light at an intensity of at least 7 mW/mm² and flashing light with a duration of at least 1 ms for each flash.

### BRIEF DESCRIPTION OF THE DRAWINGS

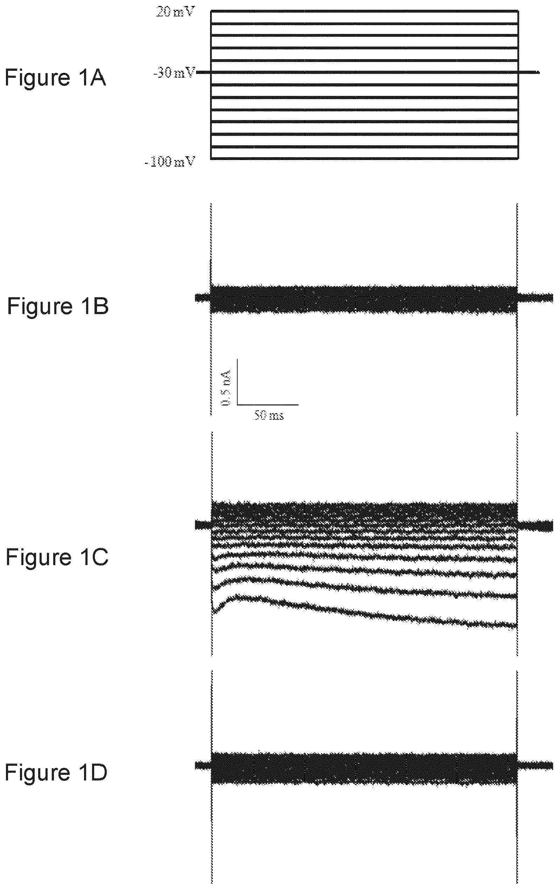

**Figure 1** demonstrates a whole cell patch clamp performed at a holding potential of -30 mV. The voltage-clamp protocol included 250ms long voltage pulses within the range of -100mV to +20mV and increments of 10mV (A). Currents were measured in darkness (B) following exposure to 470nm light (C), and again in complete darkness (D).

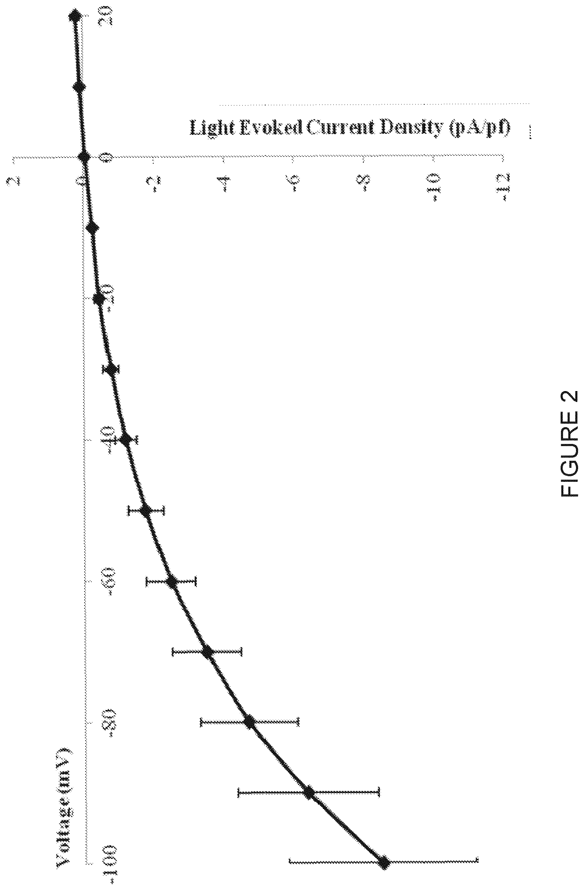

**Figure 2** shows light evoked current density plotted against holding potentials (n=7). Results are presented as mean ± standard error.

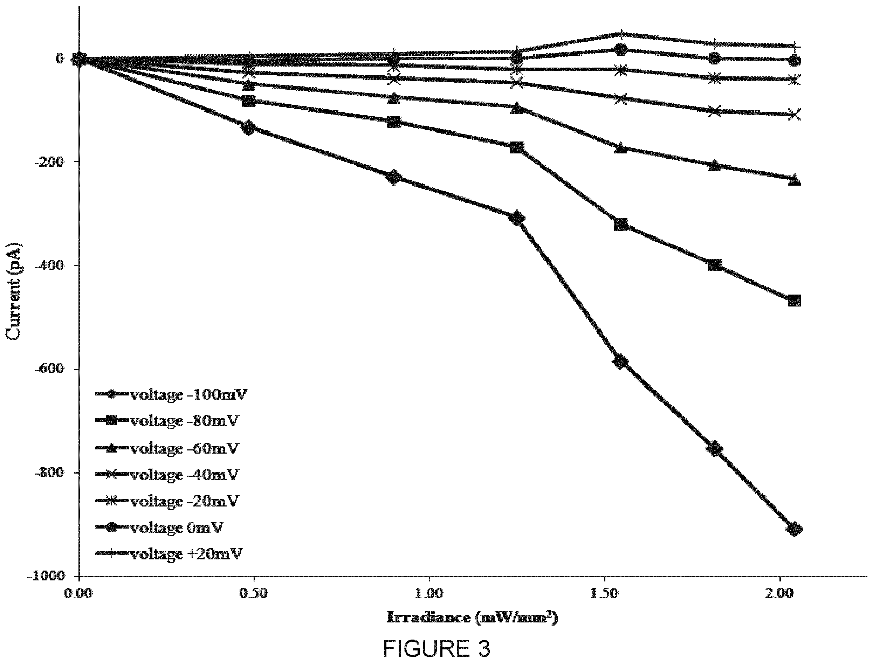

**Figure 3** exhibits the association between LED output and light evoked current density measured at different holding voltages (-100mV to 20mV, with increments of 20mV).

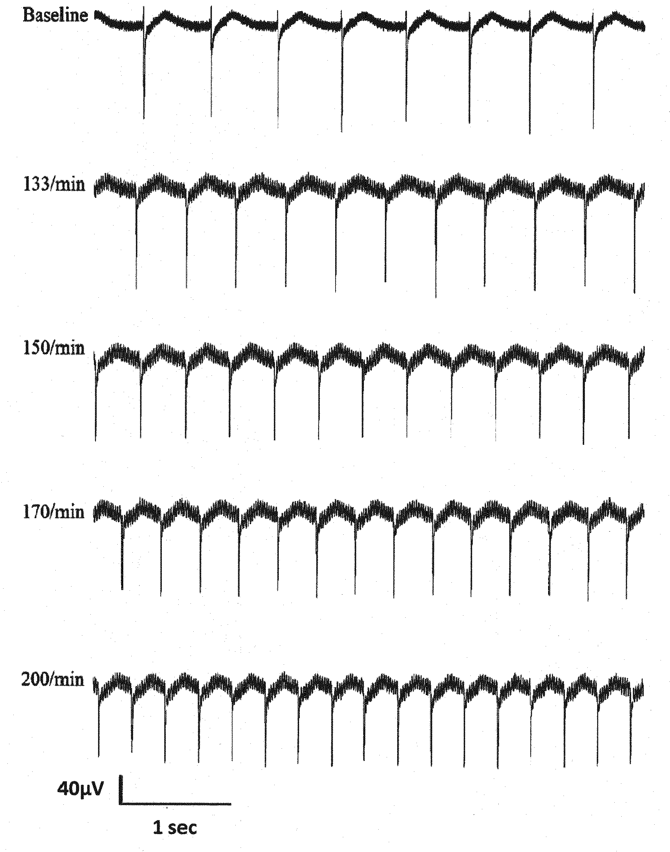

**Figure 4A** presents the electrical activity from a co-culture of NRCM and ChR2 transfected NIH 3T3 fibroblasts: baseline beating (mean rate of about 102.8 contractions/min), inter-light flash interval of 450 ms (pacing rate of 133 contractions/min), 400 ms (pacing rate of 150 contractions/min), 350 ms (pacing rate of 170 contractions/min) and 300 ms (pacing rate of 200 contractions/min).

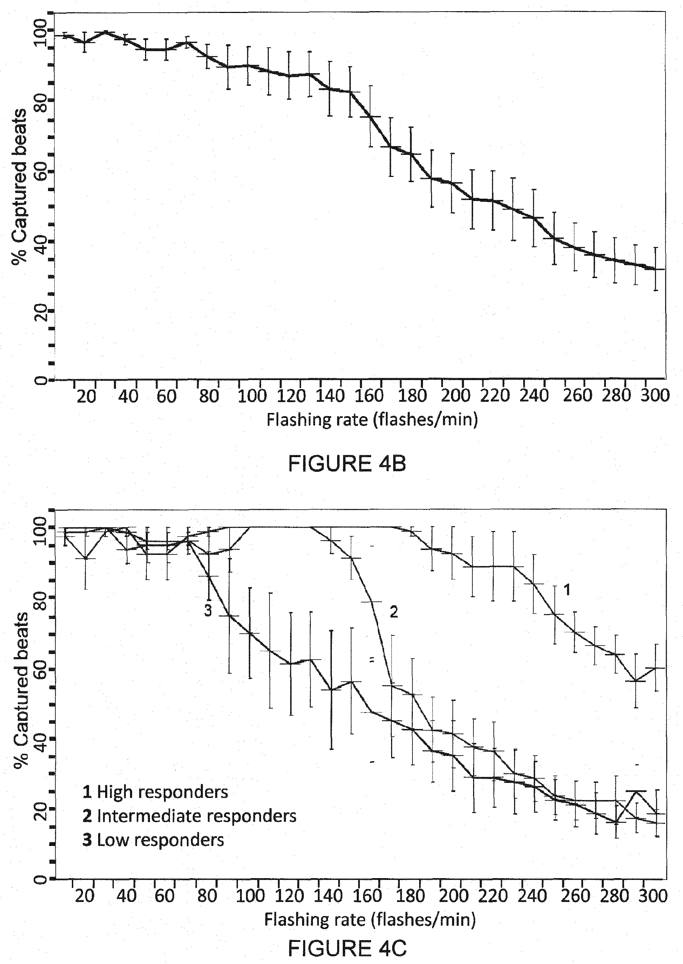

**Figure 4B** demonstrates the mean ± standard error percentage of captured beats in response to different flashing frequencies (n=15, constant flash duration of 50ms, and constant LED output of 26 mW/mm², 16 consecutive flashes for each cycle).
**Figure 4C** represents mean ± standard error response rate for high flash-rate responders (Curve no. 1; 100% flash capture rate at 170 flashes/min, n=5), intermediate responders (Curve no. 2; 100% capture rate at 120 flashes/min, n=5), and low responders (Curve no. 3; less than 100% capture rate at 120 flashes/min, n=5).

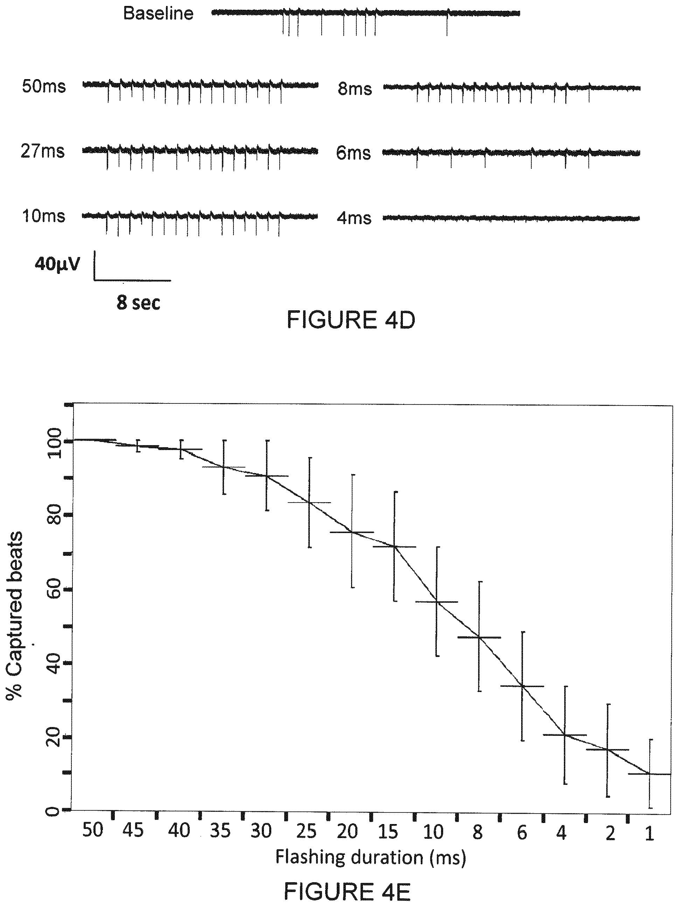

**Figures 4D** and **4E** demonstrate cardiomyocytes baseline beating rate, beating rates at various light durations (4D, 4ms to 50 ms) and the association between flash duration (50ms to 1 ms) and induced activation (4E, n=8, unchanged LED output of 26 mW/mm², and a flashing rate of 50 flashes/min).

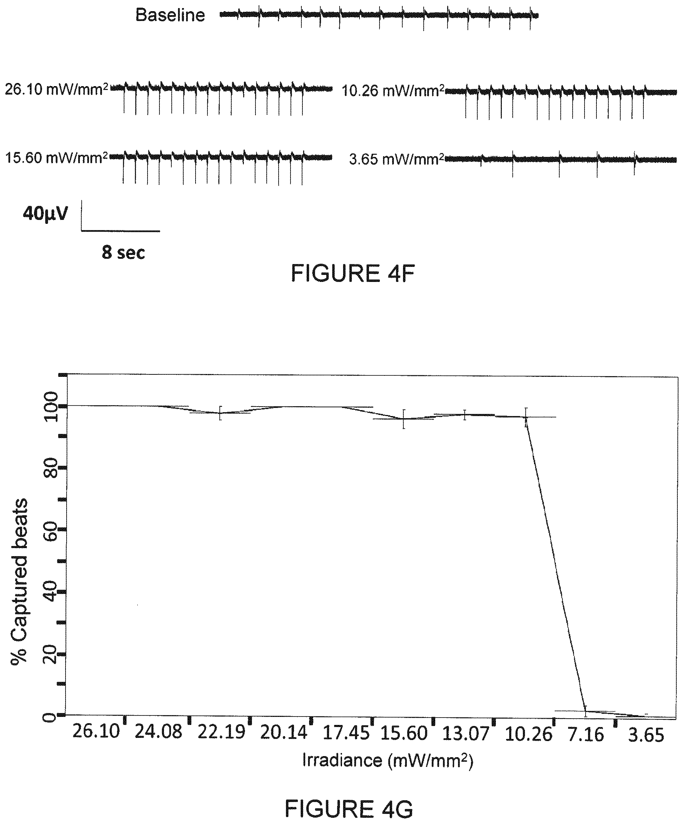

**Figures 4F** and **4G** demonstrate cardiomyocytes baseline beating rate, beating rates at various light flash intensities (4F, 3.65mW/mm² to 26.10mW/mm²) and the association between flash intensity and captured beat rates (4G, n=8, unchanged flash duration of 50ms, and a flashing rate of 50 flashes/min).

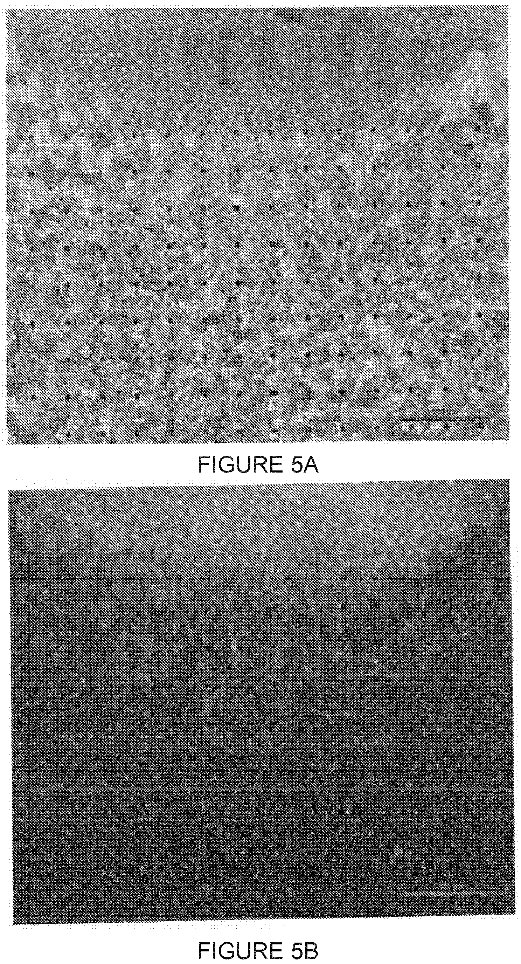

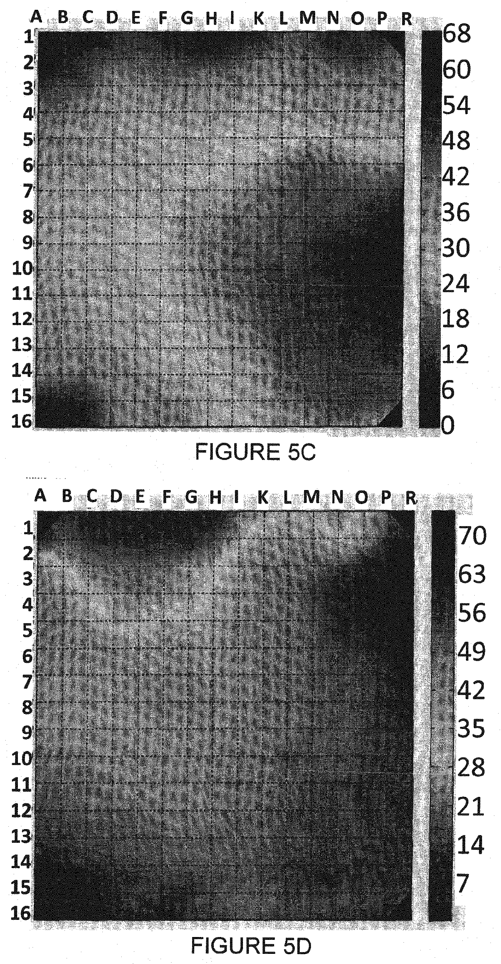

**Figure 5** shows a co-culture of NRCM and fibroblasts scattered on MEA electrodes (A) where the NIH 3T3 fibroblasts transfected with a plasmid encoding for ChR2 were seeded on the upper end of the plate (indicated by green fluorescence corresponding to the light dots in panel; B). Spontaneous activation of the culture is exhibited at the mid-area of the MEA plate (C), while light induced activation is exhibited at the area in which the transfected fibroblasts were seeded (D).

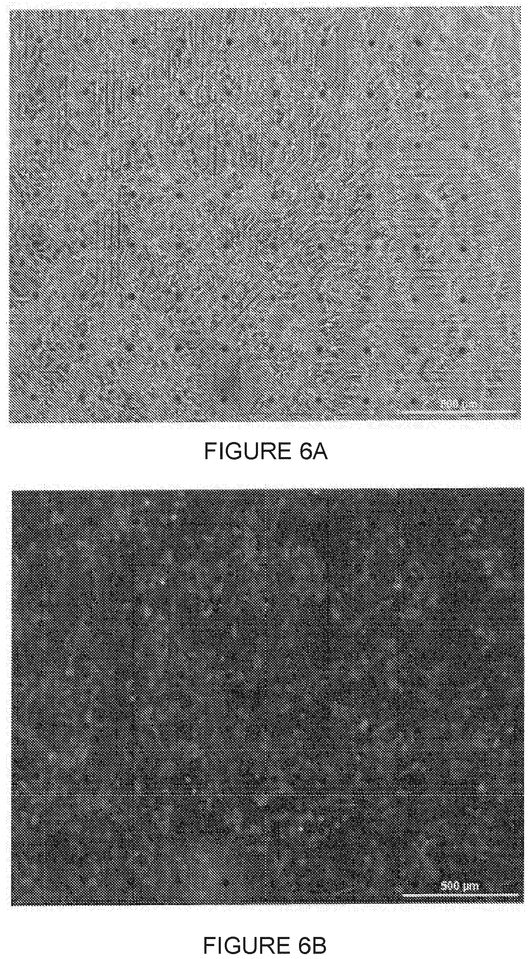

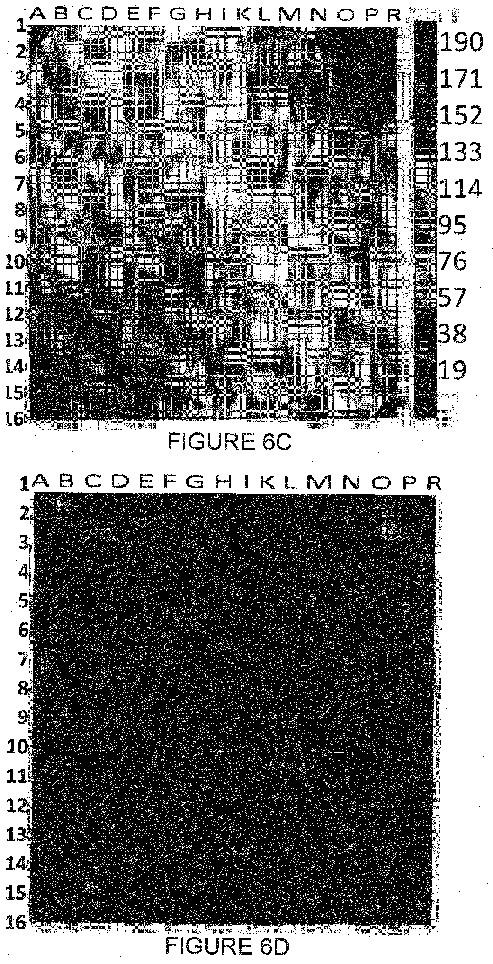

**Figure 6** presents co-culture of NRCM and NIH 3T3 fibroblasts (A) scattered on MEA electrodes, transfected with the plasmid encoding for ChR2, fused with GFP protein (B, brighter dots). Local activations maps of the co-culture are constructed from spontaneous beats (C, activation time 195.9 ms), or from light-induced beats (D, activation time 47.3 ms).

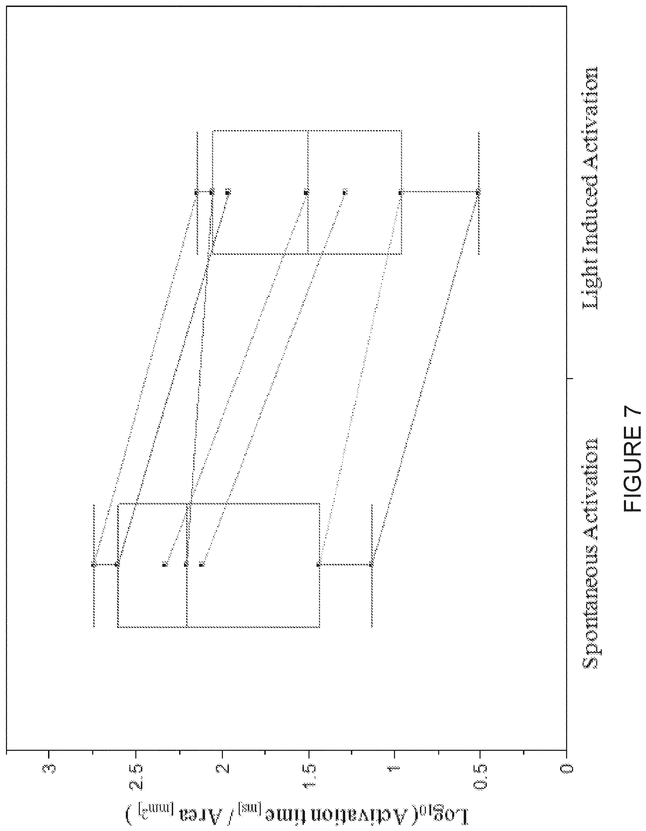

**Figure 7** is a box-plot of Log10 ratios of the corrected activation time (ms) corrected to area (mm²) during spontaneous activation, and light induced activation. Matched pairs (corresponding to the same culture) are inter-connected by a line. The black dots represent raw values. In these and subsequent box plots, the central line represents the distribution median; the box spans from 25 to 75 percentile points.

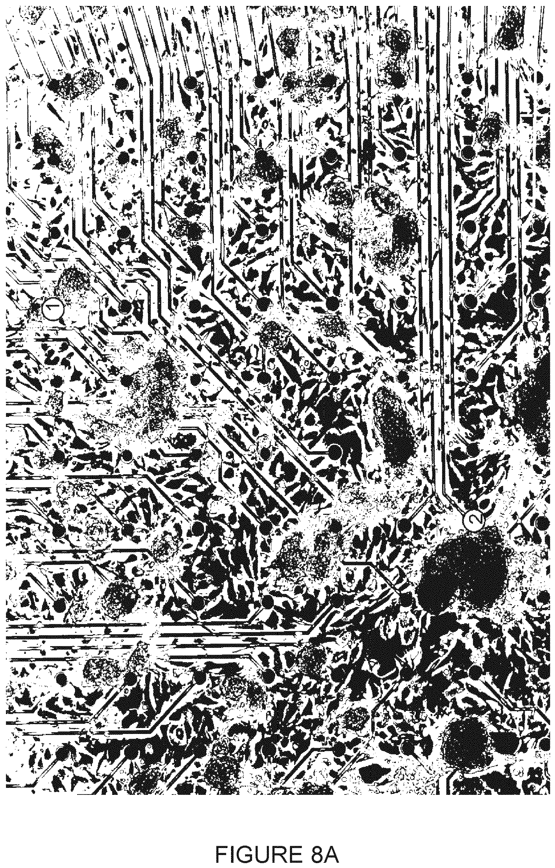

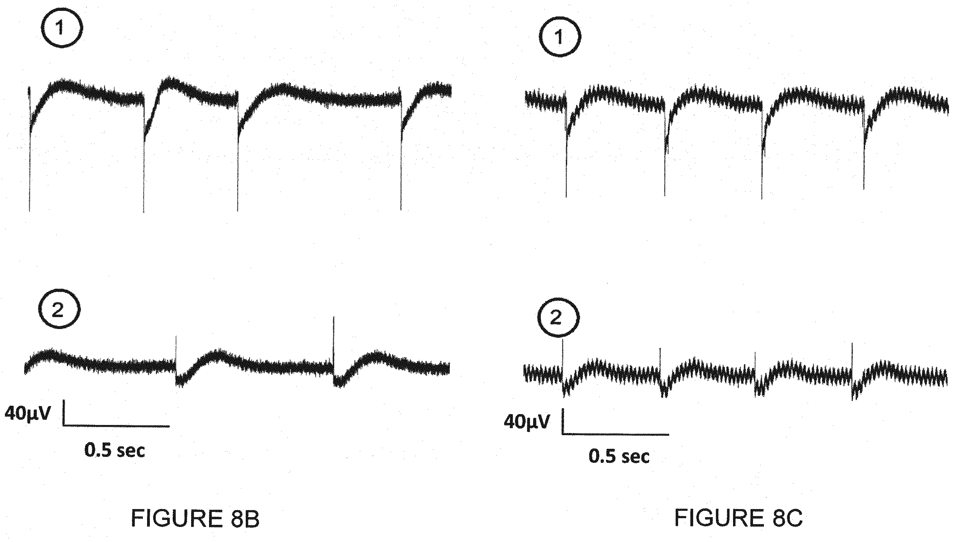

**Figure 8** presents a co-culture of NRCM and ChR2 transfected NIH 3T3 fibroblasts including two electrodes 1.34mm apart from one another (A, circled 1 and 2 at upper and lower parts of the panel, respectively). Spontaneous contraction revealed unsynchronized beating (B), while flashing every 450 ms caused synchronized and rapid contractions (C).

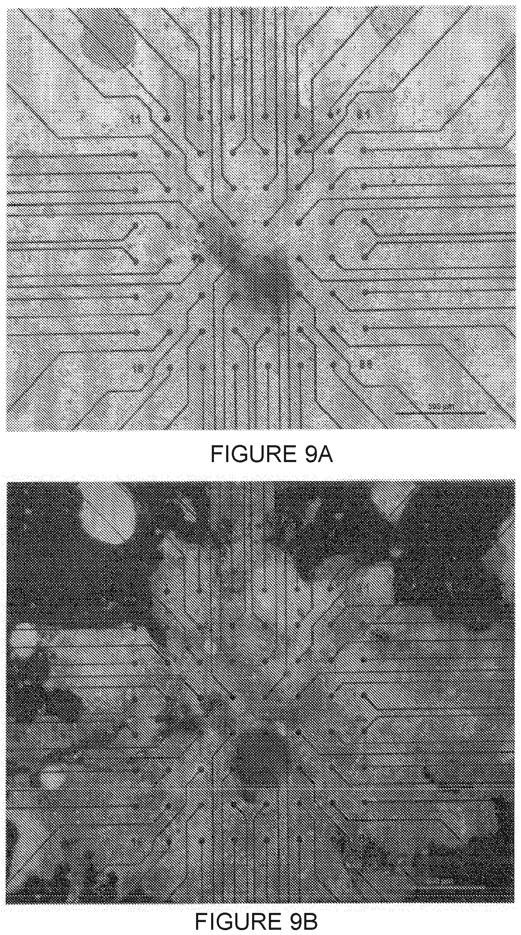

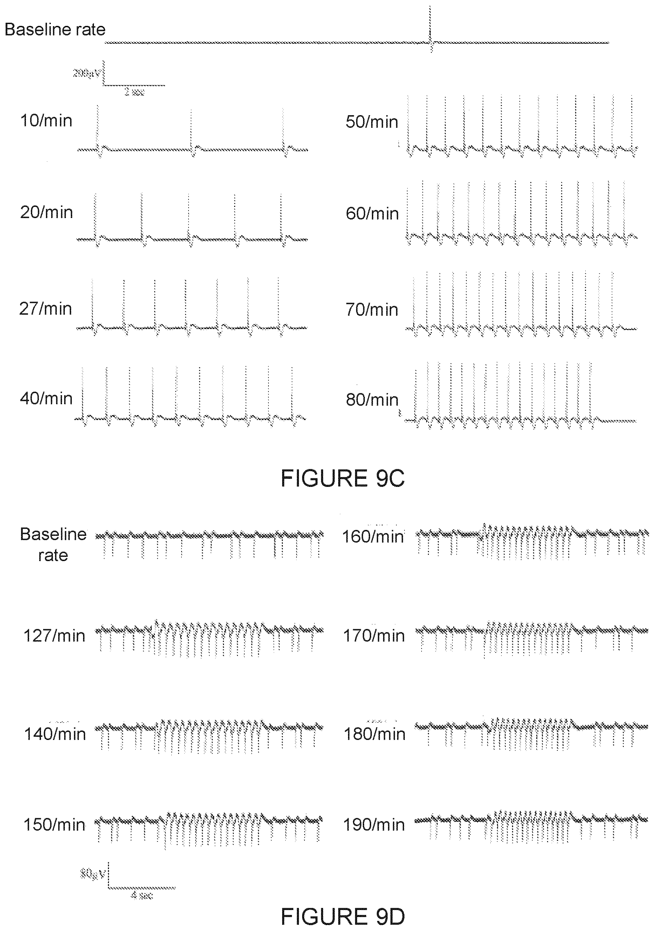

**Figure 9** demonstrates CMs driven hESCs co-cultured with NIH 3T3 AAV ChR2 fibroblasts (A), where the presence of the ChR2 channel is indicated by the bright area in the panel, corresponding to green fluorescence (B). Contraction rates of a first co-culture (from 10 to 80 flashes/min with increments of 10 flashes/min, compared to baseline contraction of about 2 contractions/min) (C) is exhibited next to the contraction rates of a second co-culture (D) at different flashing frequencies (130/min. to 190/min. compared to a spontaneous irregular contraction of about 82 contractions/min.)

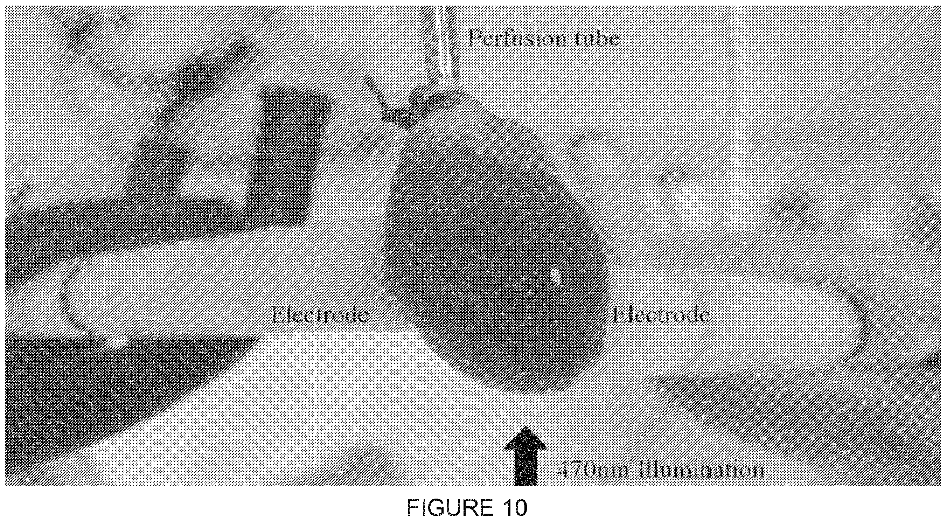

**Figure 10** presents an isolated heart, excised from a mouse two weeks after injection of AAV9 vector into the apical region of the heart (arrow), perfused with oxygenized Tyrod's solution in a langendorff perfusion system, having ECG/pacing electrodes are connected to the heart on both sides.

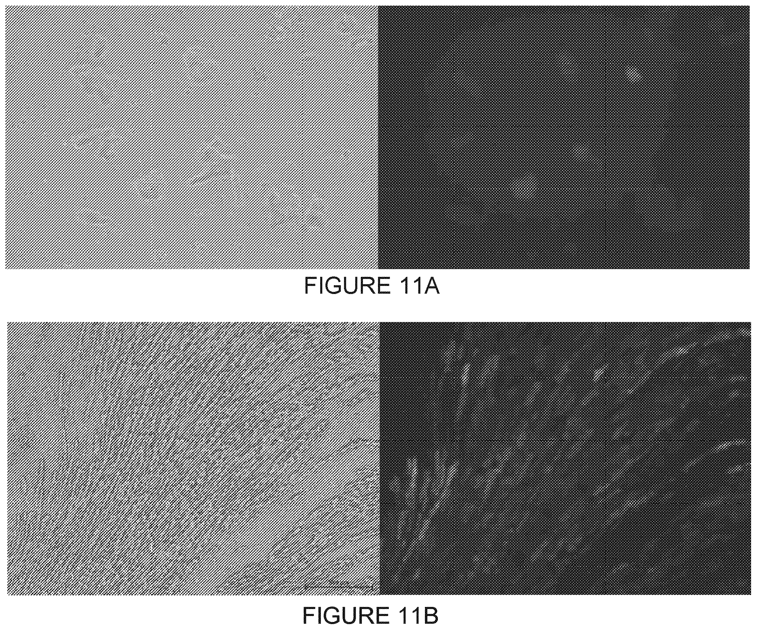

**Figure 11** presents cardiac cells extracted and isolated from a heart infected with an AAV virus encoding for the ChR2 gene (A). The presence of the ChR2 protein is indicated with the green fluorescence (bright/grey spots within the dark panel). A frozen cross section of the heart demonstrates the presence of cardiomyocytes expressing GFP protein at the injection site (B, (bright/grey areas within the dark panel)).

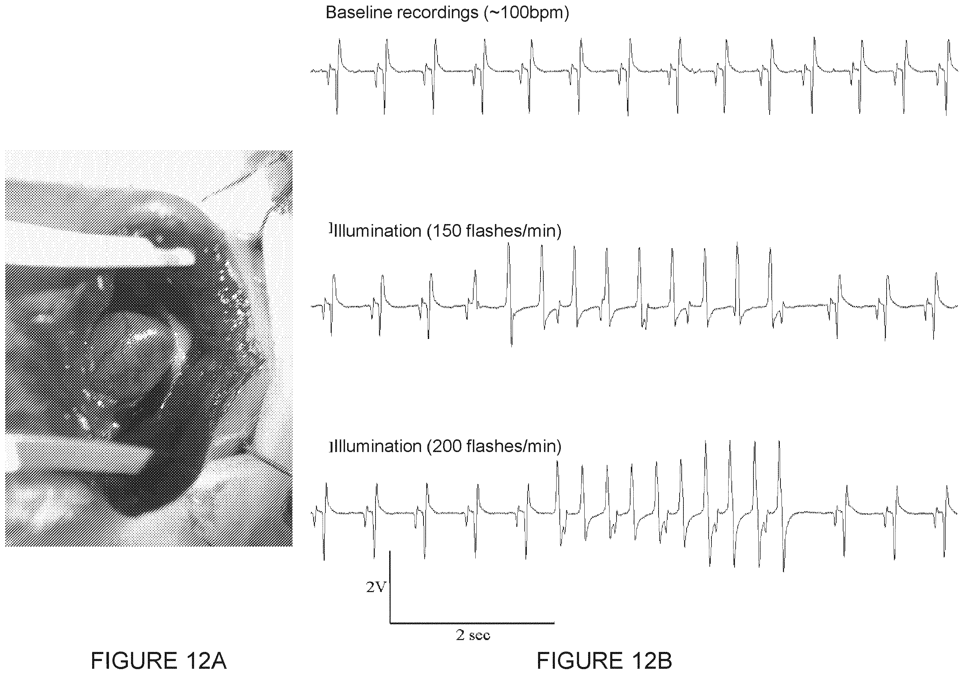

**Figure 12** presents a heart exposed following mid-thoracotomy (A) and the corresponding ECG (B) revealing a baseline beating rate of ∼100 bpm, which increased to 150 and 200 bpm following flashing at corresponding rates (B).

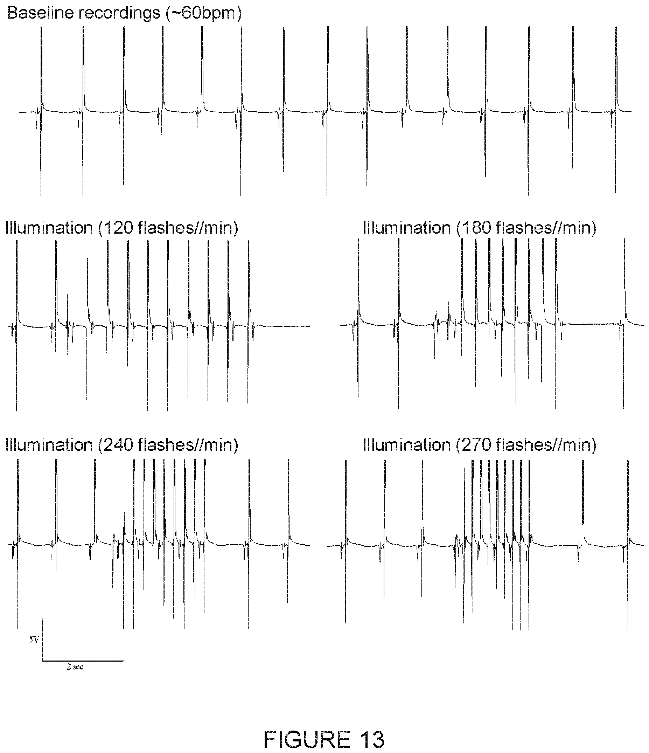

**Figure 13** presents light-induced pacing in variable rates starting from a spontaneous rate of -60 bpm, up to 300 bpm during rapid flashing, where some of the QRS are followed with retrograde P waves.

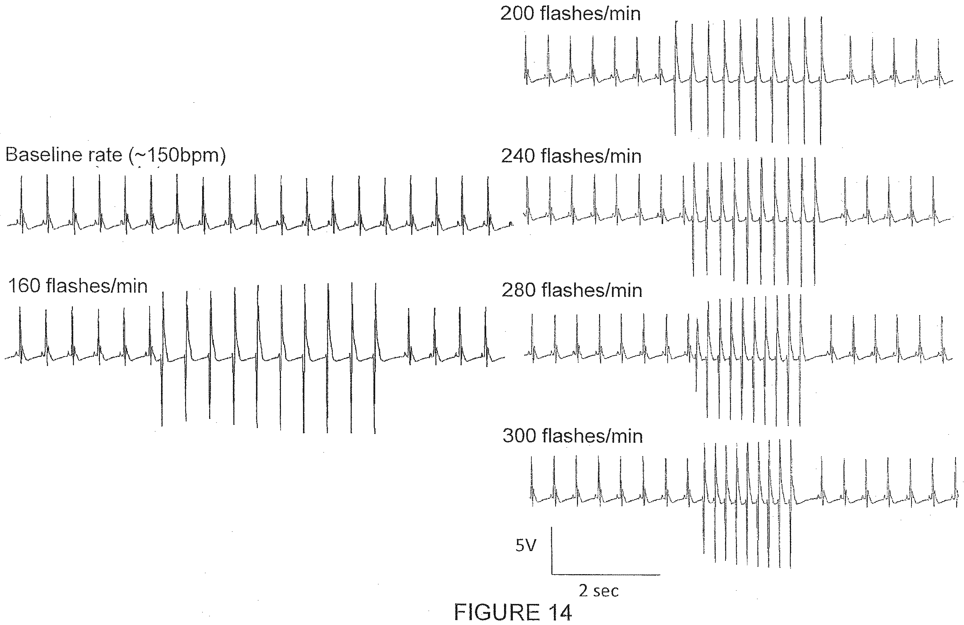

**Figure 14** presents a beating rate increase from a spontaneous rate of -150 bpm, up to 300 bpm during rapid flashing.

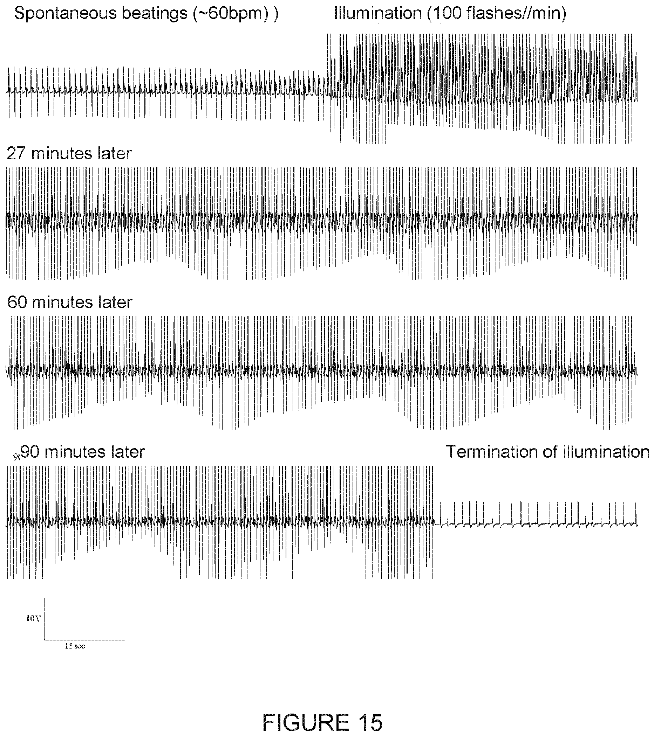

**Figure 15** presents prolonged light-induced pacing starting from a baseline rate of -60 bpm and going up to 100 bpm where each light flash resulted in a contraction during the 90 min of pacing period. Following termination of illumination spontaneous beating rate activity was recorded.

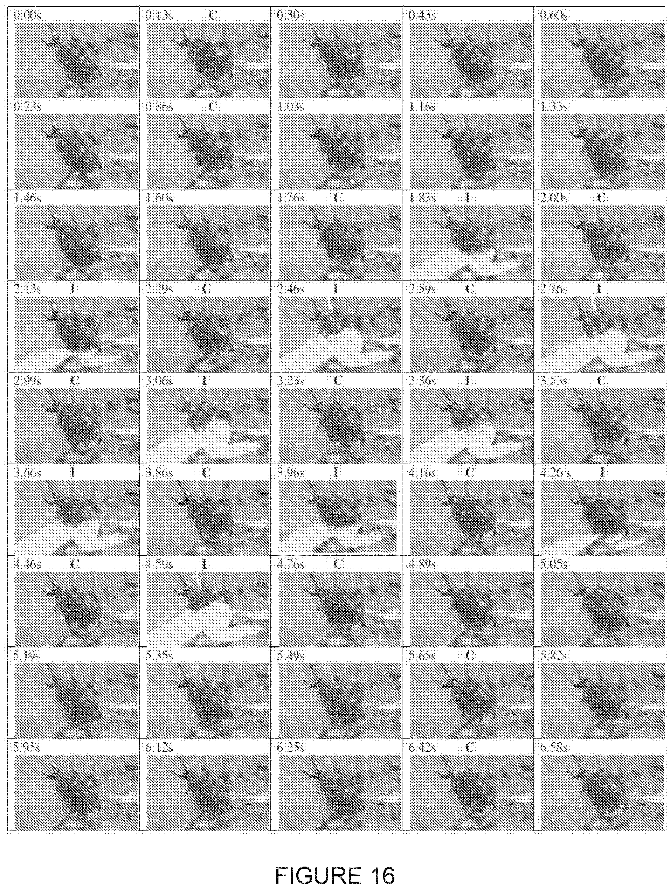

**Figure 16** presents a time sequenced, consecutive frames from a movie illustrating cardiac contraction prior to, during and following illuminations. The frames were sampled every 0.13-0.17 seconds, as indicated. Frames depicting contraction and illumination are marked C and I, respectively. The beating rate increases from a spontaneous beating rate of ∼75bpm to 200 bpm during the illumination period.

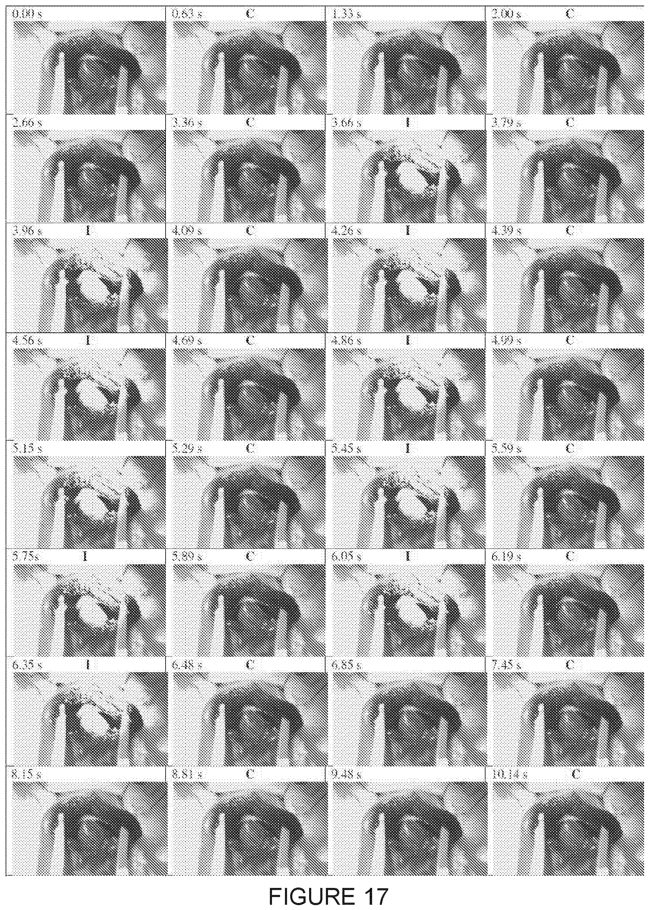

**Figure 17** presents a time sequenced, consecutive frames extracted from a movie illustrating in-vivo cardiac contraction prior to, during and following illuminations. The frames were sampled every 0.13-0.73 seconds, as indicated. Frames depicting contraction and illumination are marked C and I, respectively. The beating rate increases from a spontaneous beating rate of ∼45bpm to 200 bpm during the illumination period.

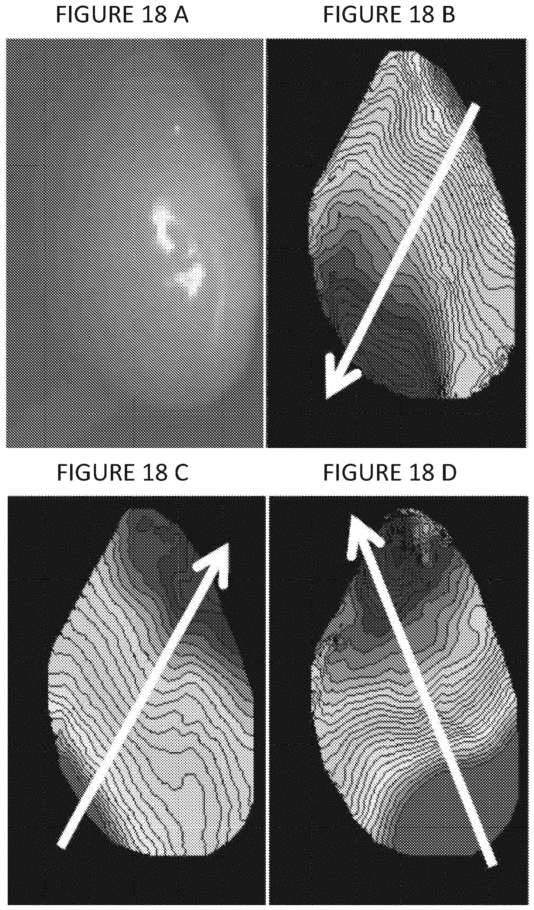

**Figure 18** presents optical activation maps of a heart injected with the AAV-ChR2 at the apex. The heart is positioned next to a pacing electrode at its upper right side and lower left side (A). Activation maps of electrical pacing initiating from the electrode positioned in the right upper side (B), the electrode positioned in the lower left side (C) and light-induced pacing initiated from the apex, where the virus was injected (D).

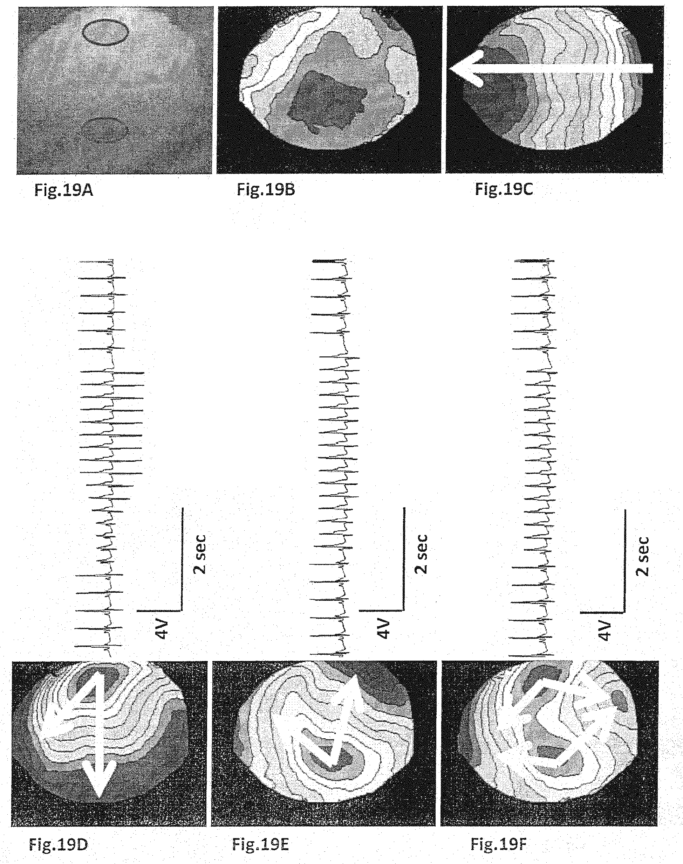

**Figure 19** presents activation maps and ECG recording. The AAV-ChR2 was injected into two different sites at the transverse axis, adjacent to the left and right ventricles (approximated injections sites are marked in circles; A) Activation maps and ECG recording were conducted for spontaneous beating (B), apical electrical pacing (C), left ventricular optical pacing (D), right ventricular optical pacing (E) and biventricular optical pacing (F). The different light-induced cardiac activation patterns produce three different electrocardiographic recordings (D-F).

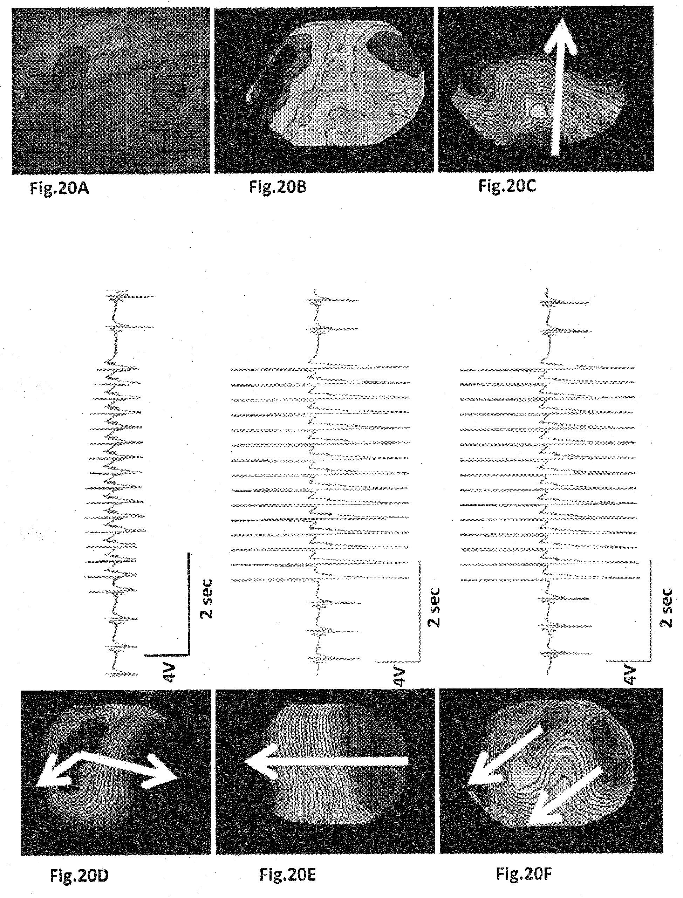

**Figure 20** presents activation maps and ECG recording. The AAV-ChR2 was injected to the apex and to left ventricular anterior wall (approximated injections sites are marked in circles; A). Activation maps and ECG recording were conducted for spontaneous beating (B), right ventricular electrical pacing (C), mid-ventricular optical pacing (D), apical optical pacing (E) and dual-sites left ventricular pacing (F). The different light-induced cardiac activation patterns produce three different electrocardiographic recordings (D-F).

### DETAILED DESCRIPTION OF THE INVENTION

The present invention provides methods, uses, kits and pharmaceutical compositions for treating heart conditions, particularly, aberrant, irregular, suppressed or non-simultaneous contraction of the heart. The methods of the invention may be applied at once, or sequentially, at one or more specific sites or areas within the heart, yet affect (regulates) the activity of the entire heart. Furthermore, the methods, uses, kits and pharmaceutical compositions of the invention can, at least during illumination therapy, cure improper contraction of the heart by inducing a predetermined, synchronous heart rate.

The methods, kits and pharmaceutical compositions of the present invention may be viewed as alternatives to electrical defibrillation (since it allows "painless defibrillation"), as alternative to catheter or surgical ablation for cardiac arrhythmias (since it is basically functional and non-destructive ablation), and as alternative to drugs (since drugs act globally on the heart and are therefore associated with significant side effects and low efficacy).

Thus, in one aspect, the present invention provides a method for treating a disease or disorder associated with suppressed, slow, irregular or unsynchronized cardiac activity in a patient in need thereof, comprising the steps of introducing into at least one site of a contractile tissue in the heart of said patient a pharmaceutical composition comprising a gene encoding a light-sensitive channel; and exposing said at least one site to light thereby inducing a controlled heart electrical activity in said patient.

In another aspect the present invention provides a pharmaceutical composition comprising a gene encoding a light-sensitive channel for the treatment of a disease or disorder associated with suppressed, slow, fast, irregular or unsynchronized cardiac activity upon exposure of said composition to light after said composition is introduced into at least one site of a contractile tissue in a heart.

In yet another aspect the present invention provides a pharmaceutical composition comprising at least one cell transfected with a gene encoding a light-sensitive channel for the treatment of a disease or disorder associated with suppressed, slow, fast, irregular or unsynchronized cardiac activity, upon exposure of said composition to light after said composition is introduced into at least one site of a contractile tissue in a heart.

In yet another aspect the present invention provides a use of a pharmaceutical composition comprising a gene encoding a light-sensitive channel for the treatment of a disease or disorder associated with suppressed, slow, fast, irregular or unsynchronized cardiac activity upon exposure of said composition to light after said composition is introduced into at least one site of a contractile tissue in a heart.

In yet another aspect the present invention provides a use of a pharmaceutical composition comprising at least one cell transfected with a gene encoding a light-sensitive channel for the treatment of a disease or disorder associated with suppressed, slow, fast, irregular or unsynchronized cardiac activity, upon exposure of said composition to light after said composition is introduced into at least one site of a contractile tissue in a heart.

The term "pharmaceutical composition" as used herein refers to a composition comprising at least one active ingredient. A gene encoding a light-sensitive channel, and a cell transfected with a gene encoding a light-sensitive channel, are each considered an active ingredient.

The phrase "introducing a pharmaceutical composition comprising a gene" as used herein refers to inserting a gene into one or more cells of the patients' contractile tissues of the heart. It should be understood that said gene can be inserted alone, or carried by a DNA vector such as a plasmid or a virus. It should be further understood that said gene can be inserted without a promoter, or cloned downstream to a constant or inducible promoter. Each possibility represents a separate embodiment of the present invention. The examples provided herein are by no way limiting to the invention, and should be used for clarification only.

The term "contractile tissue" as used herein refers to any tissue, such as, a cardiac tissue and/or a cardiac muscle tissue, containing cells that are capable of contracting or causing contraction. Each possibility represents a separate embodiment of the present invention. The contractile tissue according to the present invention, includes, but is not limited to, any section of the heart, such as, natural pacemaker cells, excluding interconnecting veins and arteries.

It would be noted that the term "exposing" refers to exposing one or more sites of the patient's heart to light, wherein each one of said one or more sites is a site that includes a light sensitive channel-expressing cell (e.g. cardiomyocytes or fibroblasts) . In some embodiments, exposing to light refers to exposing one site at the heart. In other embodiments, exposing to light refers to exposing to light a plurality of sites at the heart.

The phrase "inducing a controlled heart electrical activity" as used herein refers, *inter alia,* to depolarizing at least a portion of the patient's heart contractile tissue, said depolarization sufficient to induce a heartbeat in said patient.

In some embodiments, inducing a controlled heart electrical activity is selected from the group consisting of inducing cardiomyocytes depolarization, inducing controlled pacing, inducing controlled cardiac activation, inducing synchronized pacing, inducing synchronizing conduction, and reversing blocked or slowed conduction. Each possibility represents a separate embodiment of the present invention.

In some embodiments, said disease or disorder is selected from the group consisting of asynchronous contraction of the ventricles, asynchronous contraction of the atriums, cardiac arrhythmia, bradyarrhythmia, reentrant (due to the presence of abnormal conduction) and other forms of tachyarrhythmia. Each possibility represents a separate embodiment of the present invention.

As used herein, the term "arrhythmia" is interchangeable with "cardiac arrhythmia".

In some embodiments, the terms "bradyarrhythmia" and "bradycardia" are interchangeable.

In some embodiments, said gene encodes the light-sensitive channel Channelorhodopsin-2 or an active variant, derivative or fragment thereof. Each possibility represents a separate embodiment of the present invention. Active variant, derivative or fragment of ChR2, include, but are not limited to, Channelrhodopsin-1 (ChR1), a cation-conducting channelrhodopsin from Volvox carteri (VChR1), a VChR1-ChR2 hybrid, a green-light activated ChR chimera from Chlamydomonas, Volvox ChR1's (C1V1) and any channelrhodopsin that is suitable for the method of the invention including artificial, modified and wild channelrhodopsins. Each possibility represents a separate embodiment of the present invention.

In some embodiments, said light-sensitive channel is selected from the group consisting of ChR2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T), ChR2-step function opsins, such as, ChR2(C129), ChR2(C128A), ChR2(C128S), ChR2(D156A) and ChR2(128S/156A), VChR1-step function opsins, such as, VChR1(C123S), VChR1(123S/151A) and active variants, derivatives or fragments thereof. Each possibility represents a separate embodiment of the present invention.

In certain such embodiments, said light-sensitive channel is selected from the group consisting of ChR2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T) and active variants, derivatives or fragments thereof. Each possibility represents a separate embodiment of the present invention.

In some embodiments, exposing said at least one site to light, depolarizes a plurality, i.e. two or more of cells, in said at least one site.

In some embodiments, said at least one site at said contractile tissue is selected from the group consisting of the myocardial apex, the apical region of the heart, the sinoatrial node, the atrioventricular node, the left bundle branch, the right bundle branch, the conduction system (bundle branches and His-purkinje system), the right and left atrium, the right and the left ventricular myocardium, the right atrium, the left atrium, the right ventricle and the left ventricle. Each possibility represents a separate embodiment of the present invention.

In some embodiments, said exposing said at least one site to light comprises exposing a plurality of sites to light. In some embodiments, said exposing said at least one site to light comprises exposing 2, 3, 4, 5 or more sites to light. Each possibility represents a separate embodiment of the present invention.

In certain such embodiments, said plurality of sites is exposed to light simultaneously.

In other certain such embodiments, said plurality of sites is exposed to light consecutively.

In other certain such embodiments, one part of said plurality of sites is exposed to light simultaneously, while another part in said plurality of sites is exposed to light in a consecutive manner.

Illumination of light-sensitive channels may be done internally, e.g. by an optic fiber adjacent to the heart, more specifically to said at least one site or to said plurality of sites. Illumination of these channels may also be done externally, e.g. by an optic fiber attached to the patient's chest, in proximity to said at least one site or to said plurality of sites. In case of external illumination, using "red-shifted" depolarizing channels, i.e. channels activated by light of higher wavelengths, enable greater penetration of light into the tissue and therefore the use of less light, which is important in terms of energy preservation and clinical translation.

Thus, in some embodiments, said light has a wavelength within the range of 400nm to 700nm.

In other embodiments, said light has a wavelength within the range of 500nm to 600nm. In some embodiments, said light has a wavelength within the range of 350nm to 600nm. In some embodiments, said light has a wavelength within the range of 400nm to 550nm. In some embodiments, said light has a wavelength within the range of 440nm to 5 10nm. In some embodiments, said light has a wavelength of about 460nm to 550nm.

Unless otherwise specified, the term "about" (or alternatively "around") as used herein before a numerical value "X" refers to an interval extending ± 30% from X, and optionally, to an interval extending ± 20% from X.

In some embodiments, said light is delivered at an intensity of at least 0.01 mW/mm². In some embodiments, said light is delivered at an intensity of at least 0.1 mW/mm². In some embodiments, said light is delivered at an intensity of at least 1 mW/mm². In some embodiments, said light is delivered at an intensity of at least 7 mW/mm². In some embodiments, said light is delivered at an intensity of 7 mW/mm² to 26 mW/mm² mA. In some embodiments, said light is delivered at an intensity of at least 10 mW/mm². In some embodiments, said light is delivered at an intensity of 10 mW/mm² to 26 mW/mm² mA.

In order to induce multiple, consecutive heart beats, the contractile cells of the heart must be given time to repolarize. Thus, in some embodiments, said light is a flashing light or a pulsing light, i.e. not a constant light. In other embodiments, said light is a constant light, which is exposed to said light-sensitive channel in a flashing or pulsatile manner, e.g. by a shutter. Each possibility represents a separate embodiment of the present invention.

In some embodiments, said light is a flashing light.

In some embodiments, said flashing light is delivered at a frequency of 60 flashes/min or more. In some embodiments, said flashing light is delivered at a frequency of 300 flashes/min or less. In some embodiments, said flashing light is delivered at a frequency ranging from 60 to 300 flashes/min. In other embodiments, said frequency is lower than 200 flashes/min. In yet other embodiments, said frequency is 100 flashes/min or lower. In yet other embodiments, said frequency is 70, 80, 90, 100 or 110 flashes/min or lower. Each possibility represents a separate embodiment of the present invention.

In yet other embodiments, the duration of each flash duration of said flashing light is at least 1 ms. In yet other embodiments, the duration of each flash of said flashing light is 1 to 1000 ms. In yet other embodiments, the duration of each flash of said flashing light is 1 to 500 ms. In yet other embodiments, the duration of each flash of said flashing light is 1 to 150 ms. In yet other embodiments, the duration of each flash of said flashing light is 1 to 50 ms.

The present invention further provides, in an aspect, a method for treating a disease or disorder associated with suppressed, slow, fast, irregular or unsynchronized cardiac activity in a patient in need thereof, comprising the steps of introducing into at least one site of a contractile tissue in the heart of said patient a pharmaceutical composition comprising a cell transfected with a gene encoding a light-sensitive channel; and exposing said at least one site to light thereby inducing a controlled heart electrical activity in said patient.

The phrase "introducing a pharmaceutical composition comprising a cell" as used herein refers to implanting a cell in high proximity and/or in physical contact with one or more cells of the patients' contractile tissues of the heart. It should be understood that a single cell or a plurality of said cell can be implanted. Each possibility represents a separate embodiment of the present invention. The examples provided herein are by no way limiting to the invention, and should be used for clarification only.

In some embodiments, said cell is selected from the group consisting of fibroblasts, cardiomyocytes and stem cells such as embryonic stem cells. Each possibility represents a separate embodiment of the present invention.

Being autologous, cells derived from a certain patient would not raise any compatibility issues when reintroduced to the same patients' body. Thus, in some embodiments, said cell is derived from said patient.

In some embodiments, said cell is capable of electronic coupling or fusing with said contractile tissue. Each possibility represents a separate embodiment of the present invention.

The phrase "capable of electronic coupling or fusing" as used herein refers to the ability of the introduced, light sensitive channel-transfected cells, to connect the patients' contractile heart cells in such a way that exposing the site of said cells to light would induce the patients' contractile heart cells to contract.

The terms "coupling" are interchangeable with any one or more of terms related to coupling of cells in the context of the present invention, including, but not limited to, fusing, connecting, adhering, attaching, associating with and the like.

The present invention further provides, in an aspect, a kit for treating a disease or disorder associated with suppressed, slow, fast, irregular or unsynchronized cardiac activity in a patient in need thereof, comprising a pharmaceutical composition comprising at least one cell transfected with a gene encoding a light-sensitive channel; and means for facilitating coupling or fusing said at least one cell with a contractile tissue of the heart of a subject in need thereof.

It is to be understood that means for facilitating coupling or fusing said at least one cell with a contractile tissue of a subject in need thereof include any means known in the art for carrying such procedure, including, but not limited to, the means exemplified hereinbelow.

The present invention further provides, in an aspect, a kit for treating a disease or disorder associated with suppressed, slow, fast, irregular or unsynchronized cardiac activity in a patient in need thereof, comprising a pharmaceutical composition comprising a gene encoding a light-sensitive channel; and means for facilitating transfecting at least one cell in a contractile tissue of the heart of a subject in need thereof with said gene.

It is to be understood that means for transfecting at least one cell in a contractile tissue of the heart of a subject in need thereof with said gene, include any means known in the art for carrying such procedure, including, but not limited to, the means exemplified hereinbelow.

In some embodiments, the kit further comprises a light source adapted for providing any one or more of the following light at a wavelength within the range of 350nm to 600 nm, light at a wavelength within the range of 450 to 560nm, flashing light, flashing light ranging from 60 to 300 flashes/min, light at an intensity of at least 7 mW/mm² and flashing light with a duration of at least 1 ms for each flash.

### EXAMPLES

### Example 1- Preparation of Genetically Modulated Fibroblasts

The plasmid AAV-CAG-ChR2-GFP (a fusion gene; Addgene Corp) was used for transfecting cells with ChR2. Stable transfection was achieved in NIH 3T3 fibroblasts by the jetPEI™ transfection reagent (PolyPlus transfection Inc). Transfected cells were selected according to their fluorescence level by using repeated fluorescence-activated cell sorting.

Whole cell patch-clamp was achieved as follows. NIH 3T3 fibroblasts, transfected with the ChR2 gene and cultured on fibronectin-coated cover glasses were incubated in a growth medium. Three to four days later, the cells were viewed using an inverted microscope (Eclipse Ti; Nikon Instruments Inc., Melville, NY). Fluorescence was verified and only isolated fluorescent cells were used. Whole-cell recordings were conducted at room temperature using the MultiClamp 700B and Digidata 1440A (Axon CNS, Molecular Devices, Sunnyvale, CA, United States). Currents signals were digitally sampled at 50µs (20 kHz), and low-pass filtered at 10 kHz. Patch pipettes, made from barosilicate were pulled using a Sutter Instruments horizontal pipette puller (P-1000, Sutter Instruments, Novato, CA) with a resistance of 2.5-3.5 MΩ, when filled with the pipette solution. The bath solution contained (in mM) NaCl 140, KC13, HEPES 10, glucose 10, MgCl₂ 2, and CaCl₂ 2 (pH 7.4, adjusted with NaOH).The pipette solution contained (in mM) KC1140, Na₂ATP 10, EGTA 10, HEPES 5, CaCl₂ 1, and MgCl₂ 1 (pH 7.4, adjusted with KOH). Voltage-clamp recordings from the transfected fibroblasts were performed at a holding potential of -30 mV. To study the voltage dependence of the channel, currents were elicited by pulsing the membrane potential for 250 msec, between -100mV to +20mV, in 10mV increments (Figure 1A). Recordings were accepted only when seal resistance was more than 1 GΩ and the series resistance was lower than 20 MΩ. Junction potentials were nulled before seal formation. Measurements were conducted either in complete darkness or with LED illumination (470 nm, 26 mW/mm²). Light-evoked current traces were obtained by subtracting the currents which were measured during darkness from the currents measured during the illumination, at the matching test potential. Illumination-induced steady-state currents were determined by averaging 3000 current points (150 msec) near the end of the test pulse, and normalized to cell capacitance for each cell (n=7). Illumination-induced steady-state current densities were plotted against the corresponding test potential to form a steady-state I-V curve. To study the dependence of light intensity and light-evoked currents, they were elicited by a similar protocol at different LED output currents each time (0-26 mW/mm², increments of about 6 mW/mm²). Steady-state I-V curves were plotted for each LED output.

### Example 2 - Preparation of Cardiomvocvte Monolavers and Co-cultures

Primary cultures of 0- to 1-day-old neonatal rat (Sprague-Dawley) ventricular cardiomyocytes (NRCM) were extracted. The tissue was suspended in a culture medium (F-10, 5% FCS, 5% horse serum, 100 U/mL penicillin, 100 mg/mL streptomycin) and cardiomyocytes were extracted enzymatically with RDB. 5-bromo-2'-deoxyuridine (BrdU) was used during the preparation of the cultures to reduce the replication of non-myocytic cells. Cells were then cultured on a microelectrode array culture plate at a density of 3-10 X10⁵ cells/0.5-0.8 cm². NIH 3T3 fibroblasts, either transfected with ChR2 plasmid, or non-transfected (which were used as controls) were seeded in clusters, or scattered throughout the plate (n=7).

### Example 3 - Preparation of cardiomvocvte driven hESC and co-culture

Undifferentiated human embryonic stem cells (hESC; A2T5 clone) were cultivated in suspension for 7 to 10 days as embryoid bodies (EBs) as previously described (Kehat et al., J. Clin. Invest. 2001; 108: 407-14). Beating areas, identified within the EBs after plating, were dissected and plated on 60 microelectrode array (MEA) plates (one to two EBs per MEA plate). One day later 5-10 X10⁴ transfected fibroblasts were added and co-cultured for 5-8 days until the beating EB was surrounded with an abundant layer of fibroblasts.

### Example 4 - Multielectrode Arrays

### Mapping Technique

Extracellular recordings from the cultured NRCM were analyzed by a microelectrode array (MEA) data acquisition system (Multi Channel Systems, Germany). The MEA consists of a matrix of 252 or 60 electrodes with an inter-electrode distance of 200 µm (with an approximate area of 3 mm X 3 mm for the 252 electrodes array) and a sampling rate of 20 kHz. Temperature was kept at 37.0 ± 0.1°C during measurements. NRCM co-culture voltage was measured 1-4 days following cell culturing in order to achieve synchronized electro-mechanical activity of the contractile tissue covering the electrodes. Isoproterenol (10 µM/L) was added to co-cultures in which basal contraction rate was lower than 10 contractions/min.

### Illumination of MEA experimentation

NRCM illumination - Illumination of the 252 electrodes MEA plates was achieved with a 1,000 watt quartz tungsten halogen lamp (Newport Corporation, USA), equipped with an electronic shutter was connected to a 4-channel programmable stimulus generator (STG-1004, Multi Channel Systems, Germany). A monochromic filter (470±30 nm, Chroma Technology Corp, USA) was used to determine the narrow band-pass frequency. A 50 ms long illumination was followed by a predetermined interval within the limit of 950-250 ms, corresponding to a flashing frequency of 60-200/min.

For the evaluation of the percentage of captured light-induced contractions illumination was induced with fiber-coupled 1.0A monochromic LED (470nm, Item# M470F1, Thorlab Inc.) connected to high power LED driver (Item# LEDD1B, Thorlab Inc.). Sixteen consecutive flashes were executed (with a constant inter-flash interval), corresponding to a flashing rate ranging from 10 flashes/min to 300 flashes/min (increment of 10 flashes/min per cycle, 26 mW/mm²LED output, 50 ms long illumination). The percentage of flashes yielding contraction (i.e. capture percentage) was calculated.

In order to evaluate the response to light flash duration on NRCM excitation, 16 flashes were executed (with a constant flashing rate of 50 flashes/min, 26 mW/mm² LED current output) for each cycle with flash duration. Flash duration ranges from 50 ms to 1ms (with a decrement of 5ms for each cycle until 10 ms, than with a decrement of 2 ms between flashing cycles). The percentage of captured beats was calculated.

The effect of light intensity on NRCM activation was evaluated by applying 16 flashes (with constant flashing rate of 50 flashes/min, 50 ms long illumination) with a LED current output ranging from 26 mW/mm2 to 3.6 mW/mm2 (decrements of 3.6 mW/mm2).

### CMs driven hESCs

A 200-750 ms long illumination (26 mW/mm² LED output) with inter-flash interval corresponding to different rates (10-250 flashes/min) with an increment of 10 flashes/min per cycle.

### Data Analysis

The 252-channel data from the MEA recording were analyzed by custom-made Matlab based software. Local activation time (LAT) was calculated by detecting the local maximum of the negative slope of the signal (in absolute value). To reliably detect the LAT, a low pass finite impulse response filter was applied to the input signal with a pass-band frequency of 300Hz and a stop-band frequency of 800 Hz. The LAT was detected only in regions where the 'peak-to-trough' amplitude of the QRS complex was larger than six standard deviations of the filtered signal. In addition, the negative slope was classified as a LAT only if it was less than a median threshold of the filtered signal minus four standard deviations of the filtered signal. Activation maps were thereafter created according to the detected LAT. Activation time (ms) for the electrode area were calculated and activation time of the recording area was adjusted to the measured area.

### Statistical Analysis

Data were analyzed using JMP Pro version 10.0 (SAS Institute Inc.). Results presented as mean and standard error. Distribution was evaluated for normality by the Shapiro-Wilk test. Matched pairs were compared with the paired t-test.

### Example 5 - Whole cell patch-clamp

Cell capacitance was 42.08±8.98 pf. Whole-cell recordings were performed demonstrating the channel's functionality at the single cell level. The inward currents produced as a result of light exposure are presented in Figure 1. Overall relatively negligible currents were measured from the cells at baseline, in complete darkness (Figure 1B). Following light exposure, substantially increased inward currents were measured (Figure 1C), although the effect was eradicated when light exposure was terminated (Figure 1D). Steady-state I-V curves presenting the averaged currents produced at different test potentials due to light exposure are presented in Figure 2. Steady-state current-density-voltage curves showed typical significant inward rectification, and slightly positive reversal potential. Moreover, the currents produced at test potentials were found to be associated with LED output current amplitude, as presented in Figure 3, showing that the inward current amplitude at any given LED output current amplitude was greater than its amplitude in darkness.

### Example 6 - Whole cell patch-clamp in multi-electrode array with NRCM co-culture

A linear correlation between optical flashing frequency and the captured NRCM culture beating response was found (R² = 1). Figure 4 demonstrate an increase of the cardiomyocytes beating rate in response to a rapid monochromic light flashing from about 103 contractions/min up to 200 contractions per minute and with an overall immaculate response to light stimulation. The mean percentage of captured beats (n=15, Figure 4B, pacing rate ranged from 10 to 300 flashes/min) demonstrates that at pacing rates lower than 100 flashes/min, more than 90% of the beats were captured (yielding contraction) in all the plates. The response rate was variable: some plates demonstrated a high response rate to rapid flashing, while in other plates the percentage of captured flashes was lower (Figure 4C). Specifically, in 4 out of 15 plates (26.7%), flashing rate of 200 flashes/min yielded a 100% captured capture rate. Nevertheless, in all the plates response rates for lower flashing frequency was very high. For instance, flashing rate of 70 flashes/min (a reasonably physiological resting heart rate in humans) yielded contractions following 96.7±1.6% of flashes in all plates (n=15). At a flashing rate of 130 flashes/min, that mean captured flashes was 87.5±6.3%. Figures 4D and 4E demonstrate the correlation between flash duration and beat capture percentage. A flash duration higher than 25 ms yielded a contraction following more than 90% of the flashes (n=8). In one case, flash duration of 2 ms was sufficient for causing 100% contraction capture rate. Figures 4F and 4G demonstrate the association between the LED current output, and the co-culture response to illumination. It was demonstrated that LED outputs higher than or equal to 10 mW/mm²induced a high response of the NRCM tissue (at 10 mW/mm²LED current output illumination the percentage of captured beats was 96.9±3.1%). Below the 10 mW/mm²threshold a sharp decline in successful pacing was noted, to less than 5% captured contractions following illumination (Figure 4G).

Figure 5 demonstrates a co-culture of NRVM and fibroblasts, seeded in designated areas within the plates: transfected fibroblasts were seeded in a cluster at the higher end of the plate (Figure 5A and 5B, as GFP indicates the presence of the ChR2 light-channel (corresponding to the lighter areas in the figure)) while non-transfected fibroblasts were implanted at the lower end of the culture. Spontaneous activation of the culture was found to initiate at the mid-area of the MEA plate (Figure 5C), while light induced activation was initiated from the area in which the transfected fibroblasts were seeded (Figure 5D). The action potential propagated towards the area in which the non-transfected fibroblasts were positioned, indicating that in the absence of the ChR2 channel 470 nm monochromic light does not induce depolarization. Despite a change in contraction initiation, the overall activation time of the NRCM did not substantially change.

The co-culture of NRCM and transfected fibroblasts scattered homogenously throughout the plate (Figures 6A and 6B), yielded electrical activity measured by the 252 MEA electrodes. A spontaneous contraction (activation map is presented in Figure 6C) erupted from a random spot (lower-left area) within the electrode area. In contrast, light induced contraction was associated with activation of the contractile cells from several different sites within the plate (Figure 6D), thus causing substantial shortening of the tissue activation time (i.e. from 195.9 ms to 47.3 ms).

The activation time of the co-culture was adjusted to the area covered by the measuring electrodes in each plate. The mean selected recording area was 2.17±1.16 mm². All distributions of the activation times were normal. The logarithmic result of the calculated adjusted activation time is presented in Figure 7. A significantly decreased corrected light induced activation time was found, altered from 213.1±74.1 ms/mm² to 58.2±20.9 ms/mm² (p=0.037, corresponding to a decrease of the activation time by 70.6±7.2%).

Figure 8A demonstrates a co-culture of NIH 3T3 fibroblasts transfected with the ChR2 light-channel and NRCM seeded in a non-compact manner, resulting in NRCM clusters to achieve dys-synchronization (modeling a conduction block between two contractile areas). Two electrodes, 1.34 mm apart, were randomly chosen (marked in green and red). The measured electrical activity is presented in subsequent figures. It is evident that the non-synchronized spontaneous contraction (Figure 8B) is altered into a rapid synchronized electrical activation corresponding to a flashing every 450 ms (Figure 8C).

### Example 7 - Whole cell patch-clamp in multi-electrode array with CMs driven hESCs co-culture

A precise response to alternating flashing frequency was observed in CMs driven hESCs co-cultured with NIH 3T3 AAV ChR2 fibroblasts (Figure 9A), where the presence of the ChR2 channel is indicated by green fluorescence (Figure 9B, bright area), with a response of up to 80 contractions per minute, compared with a baseline spontaneous contraction rate of about 2 contractions/min (Figure 9C). In another example, a baseline contraction rate of about 82 contractions/min increased to up to 190 light-induced contractions/min (Figure 9D). The correlation between the flashing frequency and the measured response rate was found to be of a linear order (R² = 1).

### Example 8 - Ex-vivo optical induction of Rat heart contraction

### Surgery

Rats were anesthetized (87mg/kg Ketamin, 13mg/kg Xylazine), intubated and placed on external ventilator (100% O₂, volume of 1ml/kg). Using a left thoracotomy (3-4 intercostal space) the AAV9 vector encoding the ChR2 transgene (12 x 10¹² infective units) were injected into the apical region of the heart using a 30g needle, in order to express the transgene in the cardiomyocytes. Following the operation, the animals were treated with analgesic (buprenorphine 0.03 mg/kg). All animals were treated with Ceforex 18% 0.1 ml SC.

### ECG monitoring and optical mapping studies

Twelve to fifteen days following the operation, the animals were sacrificed (IP urethane 1.6 mg/kg). Lack of reflexes were ensured. The rib-cage was exposed, and the heart was transferred to a custom-built optical mapping chamber, where it was kept perfused using a langendorff apparatus with an oxygenized Tyrod's solution (Figure 10).

Figure 11 presents cells isolated from a heart (A) where the presence of the ChR2 protein in the heart presented in Figure 10 is indicated with green fluorescence corresponding to bright spots (B).

Figure 12 presents the heart exposed following mid-thoracotomy (A) and the corresponding ECG (B) revealing a baseline beating rate of ∼100 bpm, which increased to 150 and 200 bpm following flashing at corresponding rates (B).

In order to evaluate the percentage of captured light-induced contractions, focused illumination on the area where the virus was injected, namely, the apical region of the heart, was performed with fiber-coupled monochromic LED (470 nm, Prizmatix corp.). In order to evaluate capture at different pacing frequencies, 10 consecutive flashes were delivered at a frequency ranging from 120 to 300 flashes/min (Figure 13) in 60 flashes/min intervals.

Figure 14 present an experiment similar to the experiment described above, this time in 40 flashes/min intervals, thus demonstrating the flexibility of the system. Also, prolonged pacing (for 90 min) was attempted at a flashing rate of 100 flashes/min (Figure 15).

The perfused beating heart was attached to a digital ECG data acquisition system (Biopac systems, Inc.), and the flashing effect on the electrocardiogram was evaluated (i.e. the presence of captured beats).

Also, a high-speed CCD-based optical mapping technique (Scimedia) was used to study the effects of viral infection on the local myocardial electrical properties, and on the propagation of cardiac depolarization wave. Di-4-ANBDQBS (40µL, 29.61 mMol/L) is added to the perfusate for loading prior to voltage mapping. Voltage maps is obtained with an excitation filter of 660 nm and emission long-pass filter >715 nm. Optical mapping is performed at baseline (darkness), during electrical pacing, and following monochromatic blue-light illumination.

Quantitative analysis of the optical mapping data is performed by marking activation of each optical AP at each pixel as the maximum dF/dt. This information is then used for construction of detailed isochronal activation maps. Conduction velocity vectors is analyzed in order to evaluate whether activation is initiated from the cell injection site, or from the site of illumination.

### Histology and immunohistochemistry

Hearts are frozen, sectioned and prepared for histology and immunohistochemistry.

### Example 9 - Ex-vivo optical induction of rat heart contraction

The transfected heart of Example 8, connected to langendorff apparatus, was filmed prior to, during and following illuminations. Figure 16 presents time sequenced, consecutive frames extracted from said movie. The frames were sampled every 0.13-0.17 seconds, as indicated. Frames depicting contraction and illumination are marked C and I, respectively. The beating rate increases from a spontaneous beating rate of ∼75bpm to 200 bpm during the illumination period.

As shown in Figure 16, the starting resting heartbeat is approximately 75bpm, as can be deduced by an inter-contraction time of 0.73 seconds, e.g. between the contractions depicted at 0.13 seconds and 0.86 seconds. Upon exposing the heart to light flashing at 200 flashes-per-minute (fpm), the inter-contraction time changes to 0.3 seconds, e.g. between the contractions depicted at 3.06 seconds and 3.36 seconds, which matches a beat rate of 200 bpm. After the illumination period, the heartbeat changes back to about 75bpm, as can be deduced by an inter-contraction time of 0.77 seconds, as seen between the contractions depicted at 5.65 seconds and 6.42 seconds.

Figure 16 thus provides clear and conclusive evidence for the unprecedented ability of the methods provided by the present invention to dictate a predetermined heart rate to an otherwise aberrantly contracting heart by limited, highly site-specific gene manipulation.

### Example 10 - In-vivo optical induction of rat heart contraction

### Surgery

Rat were anesthetized (87mg/kg Ketamin, 13mg/kg Xylazine), intubated and placed on external ventilator (100% O₂, volume of 1ml/kg). Using a left thoracotomy (3-4 intercostal space) the AAV9 vector encoding the ChR2 transgene (12 x 10¹² infective units) were injected into the apical region of the heart using a 30g needle, in order to express the transgene in the cardiomyocytes. Following the operation, the animals were treated with analgesic (buprenorphine 0.03 mg/kg). All animals were treated with Ceforex 18% 0.1 ml SC.

### Illumination

Twelve to fifteen days following the operation, the animals were intubated, ventilated, and mid-thoracotomy was performed. The transfected heart was filmed prior to, during and following illuminations. Figure 17 presents time sequenced, consecutive frames extracted from said movie. The frames were sampled every 0.13-0.73 seconds, as indicated. Frames depicting contraction and illumination are marked C and I, respectively. The beating rate increases from a spontaneous beating rate of ∼75bpm to 200 bpm during the illumination period.

As shown in Figure 17, the starting resting heartbeat is approximately 45bpm, as can be deduced by an inter-contraction time of 1.36 seconds, e.g. between the contractions depicted at 0.63 seconds and 2.00 seconds. Upon exposing the heart to light flashing at 200 fpm, the inter-contraction time changes to ∼0.3 seconds, e.g. between the contractions depicted at 4.99 seconds and 5.29 seconds, which matches a beat rate of 200 bpm. After the illumination period, the heartbeat changes back to about 45bpm, as can be deduced by an inter-contraction time of 1.36 seconds, as seen between the contractions depicted at 7.45 seconds and 8.81 seconds.

Figure 17 thus provides the decisive evidence for the unprecedented ability of the methods provided by the present invention to dictate a predetermined heart rate *in-vivo* to a patient with an otherwise aberrantly contracting heart. The present application thus exemplifies methods for treating human diseases, conditions and disorders stemming from or manifested by a low and/or irregular heartbeat.

### Example 11 - Electrical versus optical induction

Figure 18 presents optical activation maps of a heart injected with the AAV-ChR2 at its lower right apex. The heart is positioned next to two pacing electrodes, one at its upper right side and one at its lower left side (A). Provided are activation maps of electrical pacing initiated by the electrode positioned in the right upper side (B), electrical pacing initiated by the electrode positioned in the lower left side (C) and light-induced pacing initiated by light, originating in the apex, where the AAV-ChR2 was injected (D).

Figure 18 thus provides a direct comparison between induction of the heart by electrical means, such as the electrodes of standard pacemakers, and induction of the heart by optical means, as provided by the methods of the present application.

As clearly demonstrated in Figure 18, optical induction produces an activation map which is highly similar to those produced by standard electrodes, suggesting that the method of induction does not affect the action potential propagation throughout the heart.

### Example 12 - Dual site optical induction

Figure 19 presents activation maps and ECG recording of a heart injected with AAV-ChR2 at two different sites at the transverse axis, adjacent to the left and right ventricles (approximated injections sites are marked in circles; A). Activation maps and ECG were recorded for a spontaneous beating (B), an apical electrical pacing (C), a left ventricular optical pacing (D), a right ventricular optical pacing (E) and a biventricular optical pacing (F).

As demonstrated in Figure 19, a one-site induction such as in a spontaneous beating (B), an apical electrical pacing (C), a left ventricular optical pacing (D) and a right ventricular optical pacing (E) results in a single action potential propagating throughout the heart. In contrast, a multi-site induction such as the duel-site biventricular optical pacing demonstrated in (F) results in several action potential originating in several sites, propagating throughout the heart in different routes, thereby imposing a fast, uniform, more synchronized heartbeat. The different light-induced cardiac activation patterns produce three different electrocardiographic recordings (D-F).

### Example 13 - Duel site optical induction

Figure 20 presents activation maps and ECG recording of a heart injected with AAV-ChR2 at the apex and at the left ventricular anterior wall (approximated injections sites are marked in circles; A). Activation maps and ECG were recorded for spontaneous beating (B), right ventricular electrical pacing (C), mid-ventricular optical pacing (D), apical optical pacing (E) and dual-sites left ventricular pacing (F).

As clearly demonstrated in Figure 20, a one-site induction such as in a spontaneous beating (B), a right ventricular electrical pacing (C), a mid-ventricular optical pacing (D) and an apical optical pacing (E) results in a single action potential propagating throughout the heart. In contrast, a multi-site induction such as the dual-sites left ventricular optical pacing demonstrated in (F) results in several action potential propagating throughout the heart, thereby imposing a fast, uniform, more synchronized heartbeat. The different light-induced cardiac activation patterns produce three different electrocardiographic recordings (D-F).

Figures 19 and 20 both provide evidence to the feasibility of multi-site, light-induced pacing. Since contrary to standard pacemakers, which induce the heart by a very limited number of electrical electrodes, the number of activation sites for light-induced pacing is substantially unlimited, thereby allowing intricate transformation/induction patterns to be tailored according to a patient's specific condition.

### Example 14 - Optogenetics for cardiac pacing

Surgical approach: The animals (rats and/or pigs) are anesthetized, intubated and placed on external ventilator. In the gene therapy approach, using a left thoracotomy (or in the pigs potentially also during endocardial or coronary catheterization) the AAV9 vector encoding the ChR2 transgene (12x10¹² infective units) is injected into the myocardial tissue (for example the apex region). In the control group, a similar protocol is used to deliver the AAV9 vector encoding only for eGFP. In the combined cell/gene therapy approach, the engineered cells (ChR2-fibroblasts) or control eGFP-fibroblasts are injected into the myocardium.

Optogenetics pacing and ECG monitoring: two weeks following in vivo cell/gene delivery, the ability to optogentically pace the animals is evaluated acutely by two approaches; (1) by in vivo setting using a mid-sternotomy open-chest approach (rats) or in pigs potentially also during endocardial/epicardial catheterization; and (2) for a more detailed investigation using the isolated heart Langendorff approach. In the latter the hearts are transferred to a custom-built optical mapping chamber, and are perfused using a Langendorff apparatus with oxygenized Tyrode's solution.

For optogenetics pacing, focused illumination to the area of interest as well as to control remote myocardial sites is performed with a fiber-coupled monochromic LED (470nm, Prizmatix) system. Initially, the ability to optogenetically pace the heart in response to delivery of flashes of monochromatic light is evaluated. The heart is connected to a digital ECG data acquisition system enabling to evaluate the ability of the illumination signals to capture (pace) the heart in the electrocardiogram. In order to evaluate capture at different pacing frequencies, ten consecutive flashes are delivered at a frequency ranging from 60 to 300 flashes/min. Finally, prolonged pacing is attempted to determine the sustainability of the effect using a fixed flashing rate of 100 flashes/min.

Optical mapping studies: A high-speed CCD-based optical mapping technique (Scimedia) is used to study the electrical activation of the heart during the different optogenetics approaches tested. The heart is transferred to a custom-built optical mapping chamber 2 weeks following cell/transgene delivery. The voltage-sensitive dyes (Di-4-ANEPPS or Di-4-ANBDQBS) are added to the perfusate for loading prior to voltage mapping. Voltage maps are obtained with an excitation filter of 485nm and emission long-pass filter >600nm (or an excitation filter of 660nm and emission long-pass filter >715nm).

Optical mapping is performed at baseline (darkness) during both spontaneous electrical activation (sinus) and during electrical pacing (by a pacing electrode) and during the application of flashes of monochromatic blue-light. The resulting optical mapping data is analyzed as dynamic displays (movies) demonstrating the propagation of the activation wave-front. Quantitative analysis of the aforementioned optical mapping data is also performed by measuring the timing of the electrical activation as the maximum dF/dt (the timing of phase 0 in the optical action potential) at each pixel. This information is used for construction of detailed isochronal activation maps. This analysis allows to determine the source of the activation wave-front during each beat as well as the resulting conduction pattern.

Electroanatomical Mapping: In the pigs studies an electrophysiological three-dimensional mapping system is used to determine the source of the electrical activation during the different rhythms (sinus, electrical pacing, optogenetics pacing)

### Example 15 - Optogenetics for cardiac resvnchronization therapy

Multisite (or diffuse) delivery of the ChR2 transgene (via the direct gene delivery or by transplantation of the ChR2-expressing cells) to allow multisite optogenetics pacing as a novel CRT strategy is applied. This optogenetics-based "biological CRT approach" includes activation of multiple cardiac sites simultaneously using a diffuse light-source. This technique is advantageous compared to current CRT approaches which are limited to activation of the heart only in a finite number of sites and at predefined locations. The methods of delivering the ChR2 transgene or cell engraftment are performed as described above in multiple sites.

A model of mechanical dyschynchrony is derived in the animals by electrical pacing of the right ventricle. This results in a left bundle branch block (LBBB)-like electrical activation pattern of the left ventricle and mechanical dysnchronization. Alternatively, the left bundle branch is ablated in order to cause chronic LBBB.

The left ventricular electrical and mechanical function during different pacing scenarios is evaluated through any one or more of the following parameters: (1) sinus rhythm, (2) right ventricular pacing (leading to LBBB and mechanical dyscynchrony), (3) optogenetics pacing from a single ventricular site, (4) optogenetics pacing from two ventricular sites, (5) optogenetics pacing from three or four ventricular sites, (6) diffuse optogenetics pacing involving multiple ventricular sites.

The total activation time and conduction pattern as well as the mechanical efficacy of the cardiac contraction are evaluated in each scenario using multiple modalities: (1) body-surface electrocardiogram (ECG), (2) optical mapping, (3) electroanatomical mapping, (4) echocardiography, (5) pressure measurements. These studies provide the optimal technique for shortening ventricular activation time and optimizing cardiac contraction and mechanical activity.

### Example 16 - Optogenetics for prevention or treatment of ventricular arrhythmias

A rat model of post-myocardial infraction ventricular tachycardia (post-MI VT) is based on a brief coronary occlusion (30min) period followed by reperfusion. This leads to a clinically-relevant infarct showing patchy histological characteristic and heterogeneous and slow conduction at the border-zone. Using this model, a stable VT circuits is induced by programmed electrical stimulation (PES) in 70% of the Langendorff-perfused hearts studied. The VT episodes are reentrant, stable, and utilize the critical slow conducting channels at the infarct border-zone. A similar model in pigs also allows pigs to be paced on coronary occlusion and reperfusion.

Synchronization of the infarct border-zone to prevent/terminate VT: Following establishment of the chronic-infarct VT model, the AAV encoding for the ChR2 protein and engineered cells expressing the ChR2 transgene are used for simultaneous activation of the border-zone, thereby synchronizing local electrical activity and refractoriness and preventing or stopping reentry. The control groups includes delivery of AAV virus encoding for GFP protein or eGFP-expressing engineered cells to the infarct border-zone of an animal model for myocardial infarction (MI). The study groups include injection of AAV-ChR2 or engineered cells expressing the ChR2 transgene to the infarct border-zone of the MI animal model.

Detailed optical mapping (or electroanatomical mapping in larger animals), programmed electrical stimulation, and histology studies are performed in the engrafted and control animals. Maps are acquired during ventricular epicardial bipolar pacing with cycle lengths from 250 to 90 msec (decremented by 10msec) and then with S1-S2 using a basic pacing cycle length (PCL) of 200 msec and S2 decremented by 10msec. Following this, program stimulation with up to S4's and burst pacing from 90 to 60 msec (decremented by 2 msec) are performed in order to induce ventricular arrhythmias. Non-sustained VT is defined as two or more ventricular beats lasting less than 30 seconds. VT inducibility is defined as sustained VT or ventricular fibrillation (VF) lasting ≥30 seconds. Simultaneous to the electrical stimulation, illumination of the borderzone with 470nm irradiance is used, thereby resynchronizing activation of the borderzone, and preventing VT induction.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.
Aspects of the invention, corresponding to the original claims of PCT/IL2013/050893 (WO 2014/068568), include the following:
1. A method for treating a disease or disorder associated with suppressed, slow, irregular or unsynchronized cardiac activity in a patient in need thereof, comprising the steps of:
   (a) introducing into at least one site of a contractile tissue in the heart of said patient a pharmaceutical composition comprising a gene encoding a light-sensitive channel; and
   (b) exposing said at least one site to light, thereby inducing a controlled heart electrical activity in said patient.
2. The method of aspect 1, wherein inducing a controlled heart electrical activity is selected from the group consisting of inducing cardiomyocytes depolarization, inducing controlled pacing, inducing controlled cardiac activation, inducing synchronized pacing, inducing synchronizing conduction, and reversing blocked or slow conduction.
3. The method of aspect 1, wherein said disease or disorder is selected from the group consisting of asynchronous contraction of the ventricles, asynchronous contraction of the atriums, cardiac arrhythmia, bradyarrhythmia, reentrant and tachyarrhythmia.
4. The method of aspect 1, wherein said light-sensitive channel is selected from the group consisting of Channelorhodopsin-2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T), ChR2-step function opsins, ChR2(C129), ChR2(C128A), ChR2(C128S), ChR2(D156A), ChR2(128S/156A), VChR1-step function opsins, VChR1(C123S), VChR1(123S/151A) and active variants, derivatives or fragments thereof.
5. The method of aspect 1, wherein said light-sensitive channel is Channelorhodopsin-2.
6. The method of aspect 1, wherein said at least one site at said contractile tissue is selected from the group consisting of the myocardial apex, the apical region of the heart, the sinoatrial node, the atrioventricular node, the left bundle branch, the right bundle branch, the conduction system (bundle branches and His-purkinje system), the right and left atrium, the right and the left ventricular myocardium, the right atrium, the left atrium, the right ventricle and the left ventricle.
7. The method of aspect 1, wherein said exposing said at least one site to light comprises exposing a plurality of sites to light.
8. The method of aspect 7, wherein said plurality of sites are exposed to light simultaneously.
9. The method of aspect 7, wherein said plurality of sites are exposed to light consecutively.
10. The method of aspect 1, wherein said light has a wavelength within the range of 350nm to 600nm.
11. The method of aspect 1, wherein said light is delivered at an intensity of at least 7 mW/mm².
12. The method of aspect 1, wherein said light is a flashing light.
13. The method of aspect 12, wherein said flashing light is delivered at a frequency ranging from 60 to 300 flashes/min.
14. The method of aspect 12, wherein said frequency is lower than 200 flashes/min.
15. The method of aspect 12, wherein the duration of each flash of said flashing light is at least 1 ms.
16. A method for treating a disease or disorder associated with suppressed, slow, irregular or unsynchronized cardiac activity in a patient in need thereof, comprising the steps of:
   (a) introducing into at least one site of a contractile tissue in the heart of said patient a pharmaceutical composition comprising at least one cell transfected with a gene encoding a light-sensitive channel; and
   (b) exposing said at least one site to light, thereby inducing a controlled heart electrical activity in said patient.
17. The method of aspect 16, wherein inducing a controlled heart electrical activity is selected from the group consisting of inducing cardiomyocytes depolarization, inducing controlled pacing, inducing controlled cardiac activation, inducing synchronized pacing, inducing synchronizing conduction, and reversing blocked or slow conduction.
18. The method of aspect 16, wherein said disease or disorder is selected from the group consisting of asynchronous contraction of the ventricles, asynchronous contraction of the atriums, cardiac arrhythmia, bradyarrhythmia, reentrant and tachyarrhythmia.
19. The method of aspect 16, wherein said light sensitive channel is selected from the group consisting of Channelorhodopsin-2, ChR2(H134R), ChR2(T159C), ChR2(L132C), ChR2(E123A), ChR2(E123T), ChR2(E123T/T159C), ChIEF, ChRGR, VChR1, C1V1, C1V1-ChETA(E162T), C1V1-ChETA(E122T/E162T), ChR2-step function opsins, ChR2(C129), ChR2(C128A), ChR2(C128S), ChR2(D156A), ChR2(128S/156A), VChR1-step function opsins, VChR1(C123S), VChR1(123S/151A) and active variants, derivatives or fragments thereof.
20. The method of aspect 16, wherein said light-sensitive channel is Channelorhodopsin-2 or an active variant, derivative or fragment thereof.
21. The method of aspect 16, wherein exposing the at least one site to light, depolarizes said at least one cell in said at least one site.
22. The method of aspect 16, wherein said at least one site at said contractile tissue is selected from the group consisting of the myocardial apex, the apical region of the heart, the sinoatrial node, the atrioventricular node, the left bundle branch, the right bundle branch, the conduction system (bundle branches and His-Purkinje system), the right and left atrium, the right and the left ventricular myocardium, the right atrium, the left atrium, the right ventricle and the left ventricle.
23. The method of aspect 16, wherein said exposing said at least one site to light comprises exposing a plurality of sites to light.
24. The method of aspect 23, wherein said plurality of sites are exposed to light simultaneously or consecutively.
25. The method of aspect 16, wherein said light has a wavelength within the range of 350nm to 600nm.
26. The method of aspect 16, wherein said light is delivered at an intensity of at least 7 mW/mm².
27. The method of aspect 16, wherein said light is a flashing light.
28. The method of aspect 27, wherein said flashing light is delivered at a frequency ranging from 60 to 300 flashes/min.
29. The method of aspect 27, wherein said frequency is lower than 200 flashes/min.
30. The method of aspect 27, wherein the duration of each flash of said flashing light is at least 1 ms.
31. The method of aspect 16, wherein said cell is selected from the group consisting of fibroblasts, cardiomyocytes and derivatives of stem cells.
32. The method of aspect 16, wherein said cell is derived from said patient.
33. The method of aspect 16, wherein said cell is capable of electronic coupling or fusing with said contractile tissue.
34. A kit for treating a disease or disorder associated with suppressed, slow, irregular or unsynchronized cardiac activity in a patient in need thereof, comprising a pharmaceutical composition comprising at least one cell transfected with a gene encoding a light-sensitive channel; and means for facilitating coupling or fusing said at least one cell with a contractile tissue of a subject in need thereof.
35. The kit according to aspect 34, further comprising a light source.
36. The kit according to aspects 35, wherein said light source is adapted for providing at least one of light at a wavelength within the range of 350nm to 600nm, light at a wavelength within the range of 450nm to 560nm, flashing light, flashing light ranging from 60 to 300 flashes/min, light at an intensity of at least 7 mW/mm² and flashing light with a duration of at least 1 ms for each flash.
37. A kit for treating a disease or disorder associated with suppressed, slow, irregular or unsynchronized cardiac activity in a patient in need thereof, comprising a pharmaceutical composition comprising a gene encoding a light-sensitive channel; and means for facilitating transfecting a contractile tissue of the heart of a subject in need thereof with said gene.
38. The kit according to aspect 37, further comprising a light source.
39. The kit according to aspects 37, wherein said light source is adapted for providing at least one of light at a wavelength within the range of 350nm to 600nm, light at a wavelength within the range of 450nm to 560nm, flashing light, flashing light ranging from 60 to 300 flashes/min, light at an intensity of at least 7 mW/mm² and flashing light with a duration of at least 1 ms for each flash.
40. A pharmaceutical composition comprising a gene encoding a light-sensitive channel for the treatment of a disease or disorder associated with suppressed, slow, irregular or unsynchronized cardiac activity upon exposure of said composition to light after said composition is introduced into at least one site of a contractile tissue in a heart.
41. A pharmaceutical composition comprising at least one cell transfected with a gene encoding a light-sensitive channel for the treatment of a disease or disorder associated with suppressed, slow, , irregular or unsynchronized cardiac activity, upon exposure of said composition to light after said composition is introduced into at least one site of a contractile tissue in a heart.
42. Use of a pharmaceutical composition comprising a gene encoding a light-sensitive channel for the treatment of a disease or disorder associated with suppressed, slow, irregular or unsynchronized cardiac activity upon exposure of said composition to light after said composition is introduced into at least one site of a contractile tissue in a heart.
43. Use of a pharmaceutical composition comprising at least one cell transfected with a gene encoding a light-sensitive channel for the treatment of a disease or disorder associated with suppressed, slow, irregular or unsynchronized cardiac activity, upon exposure of said composition to light after said composition is introduced into at least one site of a contractile tissue in a heart.

## Claims

1. A pharmaceutical composition comprising a cell transfected with a gene encoding a light-sensitive channel for use in the treatment of a disease or disorder associated with suppressed, slow, irregular or unsynchronized cardiac activity in a human patient upon exposure of said composition to light after said composition is introduced by direct injection into a plurality of sites of contractile tissue in the heart of said patient.

2. The pharmaceutical composition for use according to claim 1, wherein exposure of said composition to light induces a controlled heart electrical activity selected from inducing cardiomyocytes depolarization, inducing controlled pacing, inducing controlled cardiac activation, inducing synchronized pacing, inducing synchronizing conduction, and reversing blocked or slow conduction.

3. The pharmaceutical composition for use according to claim 1, wherein said disease or disorder is selected from asynchronous contraction of the ventricles, asynchronous contraction of the atriums, cardiac arrhythmia, bradyarrhythmia, post-myocardial infarction ventricular tachycardia and tachyarrhythmia.

4. The pharmaceutical composition for use according to claim 1, wherein said light-sensitive channel is Channelorhodopsin-2.

5. The pharmaceutical composition for use according to claim 1, wherein said plurality of sites of a contractile tissue are selected from the sinoatrial node, the atrioventricular node, the conduction system, the right and left atrium, and the right and the left ventricular myocardium.

6. The pharmaceutical composition for use according to claim 1, wherein said plurality of sites are exposed to light simultaneously.

7. The pharmaceutical composition for use according to claim 1, wherein said plurality of sites are exposed to light consecutively.

8. The pharmaceutical composition for use according to claim 1, wherein said light has a wavelength within the range of 350nm to 600nm.

9. The pharmaceutical composition for use according to claim 1, wherein said light is delivered at an intensity of at least 1 mW/mm².

10. The pharmaceutical composition for use according to claim 1, wherein said light is a flashing light that is delivered at a frequency ranging from 60 to 300 flashes/min.

11. The pharmaceutical composition for use according to claim 10, wherein the duration of each light impulse is at least 1 ms.

12. A kit for treating a disease or disorder associated with suppressed, slow, irregular or unsynchronized cardiac activity in a patient in need thereof, comprising a pharmaceutical composition comprising at least one cell transfected with a gene encoding a light-sensitive channel; and means for facilitating coupling or fusing said at least one cell with a contractile tissue of a subject in need thereof.

13. The kit according to claim 34, further comprising a light source.

14. The kit according to claims 35, wherein said light source is adapted for providing at least one of light at a wavelength within the range of 350nm to 600nm, light at a wavelength within the range of 450nm to 560nm, flashing light, flashing light ranging from 60 to 300 flashes/min, light at an intensity of at least 7 mW/mm² and flashing light with a duration of at least 1 ms for each flash.
